# EUROPEAN PATENT APPLICATION

(11) **EP 4 130 028 A1**
(43) Date of publication of application: **08.02.2023**
(21) Application number: 21189516.4
(22) Date of filing: 03.08.2021
(51) Int. Cl.: C07K 14/725, A61P 35/00, A61K 35/17

(54) **ENGINEERED TCR COMPLEX AND METHODS OF USING SAME**

(71) Applicant: Rhazes Therapeutics Ltd, 2020000 Shfaram (IL)
(72) Inventor: YASSIN, Muhammad, 2020000 ShefarAm (IL); SAWAIED, Muneer, 2020000 Shefa-Amr (IL)
(74) Representative: Gregson, Anna Louise

(57) **Abstract**

Engineered T cell receptor (TCR) complex and methods of using same are provided. Accordingly, there is provided a TCR complex comprising a TCR comprising a TCR alpha polypeptide and a TCR beta polypeptide, wherein said TCR is devoid of an antigen-binding domain, and a CD3 polypeptide devoid of a heterologous antigen-binding domain and wherein said TCR complex is capable of being presented on a surface of a T cell expressing same. Also provided are polynucleotides encoding the TCR, T cells expressing the TCR complex and methods of using same.

## Description

### SEQUENCE LISTING STATEMENT

The ASCII file, entitled 88166 SequenceListing.txt, created on July 24, 2021, comprising 108,099 bytes, submitted concurrently with the filing of this application is incorporated herein by reference.

### FIELD AND BACKGROUND OF THE INVENTION

The present invention, in some embodiments thereof, relates to engineered TCR complex and methods of using same.

Cancer immunotherapy, including cell-based therapy, antibody therapy and cytokine therapy, has emerged in the last couple of years as a promising strategy for treating various types of cancer owing to its potential to evade genetic and cellular mechanisms of drug resistance and to target tumor cells while sparing healthy tissues.

Cell-based therapy using e.g. T cells having a T cell receptor (TCR) specific for an antigen differentially expressed in association with an MHC class I molecule on cancer cells or having a chimeric antigen receptors (CAR) comprising an antigen recognition moiety [e.g., a single chain variable fragment (scFv)] and a T-cell activation moiety were shown to exert anti-tumor effects in several types of cancers, e.g. hematologic malignancies. However, TCRs are limited in their recognition spectrum and the MHC class and in addition, when introducing an exogenous TCR to a T cell there is a risk of hybridization between exogenous and endogenous chains, which may induce recognition of autoantigens [Van Loenen MM, et al. Proc Natl Acad Sci USA. 2010;107:10972-7]. CAR T cells on the other hand, despite their advantages, have critical flaws that need to be solved to allow for full utilization of the technology in clinical treatments, including e.g. severe side effects, vigorous expansion in the presence of heavy tumor burden leading to tumor lysis syndrome and cytokine release syndrome, development of tumor escape variants which have lost the target antigen during treatment [Morgan RA et al. (2010) Mol Ther. 18: 843-51; Brudno JN et al. (2016) Blood. American Society of Hematology; page 3321-30; and Grupp SA et al. (2013) N Engl J Med 368: 1509-18]. Further, using allogeneic T cells for therapy imposes the risk of harmful recognition of autoantigens leading to graft versus host disease (GVHD).

Antibody-based cancer immunotherapies, such as monoclonal antibodies, antibody-fusion proteins, and antibody drug conjugates (ADCs) depend on recognition of cell surface molecules that are differentially expressed on cancer cells relative to non-cancerous cells and/or immune-checkpoint blockade. Binding of an antibody-based immunotherapy to a cancer cell can lead to cancer cell death via various mechanisms, e.g., antibody-dependent cell-mediated cytotoxicity (ADCC), complement-dependent cytotoxicity (CDC), direct cytotoxic activity of the payload from an antibody-drug conjugate (ADC) or suppressive checkpoint blockade. Many of these mechanisms initiate through the binding of the Fc domain of cell-bound antibodies to specialized cell surface receptors (Fc receptors) on hematopoietic cells. In recent years another type of antibody-based therapy was suggested, namely anti-CD3 bi-specific antibodies, such as Odronextamab and Blinatumomab. These bi-specific antibodies engage CD3 positive T cells with tumor-associated antigen and thus promote T cells to attack tumor cells.

Immunotherapies combining principles of antibody-based therapy, CAR T cells and/or TCR based immunotherapy have been disclosed [see e.g. Choi BD et al. (2019) Nature Publishing Group; 37: 1049-58; Chandran and Klebanoff (2019) Immunological Reviews. 290:127-147; Benjamin R. (2020) Lancet 396: 1885-94; Liu et al. (2021) Sci. Transl. Med. 13, eabb5191; Helsen C.W et al. (2018) Nature Communications 9:3049; Baeuerle P.A. et al. (2019) Nature Communications 10:2087].

### Additional background art includes:

International Patent Application Publication Nos. WO2019222275, WO2021035170 and WO2015143224;
US Patent Application Publication Nos. US20190388472 and US20190070248;
Japanese Patent No. JP2017514471; and
Russian Patent No. RU2725542.

### SUMMARY OF THE INVENTION

According to an aspect of some embodiments of the present invention there is provided a T cell receptor (TCR) complex comprising a TCR comprising a TCR alpha polypeptide and a TCR beta polypeptide, wherein the TCR is devoid of an antigen-binding domain, and a CD3 polypeptide devoid of a heterologous antigen-binding domain; and wherein the TCR complex is capable of being presented on a surface of a T cell expressing same.

According to an aspect of some embodiments of the present invention there is provided a T cell receptor (TCR) comprising a human TCR alpha polypeptide and a human TCR beta polypeptide, wherein the TCR is devoid of an antigen-binding domain, and wherein the TCR alpha and beta polypeptides comprise amino acid modifications enabling presentation of the TCR as a TCR complex on a surface of a T cell expressing same.

According to some embodiments of the invention, the TCR comprises a heterologous dimerizing moiety.

According to some embodiments of the invention, the heterologous dimerizing moiety comprises a cysteine residue at each of the TCR alpha polypeptide and the TCR beta polypeptide.

According to some embodiments of the invention, the TCR alpha polypeptide and the TCR beta polypeptide comprise a human TCR alpha polypeptide and a human TCR beta polypeptide.

According to some embodiments of the invention, the TCR alpha polypeptide and the TCR beta polypeptide comprise a chimeric human and murine TCR alpha polypeptide and a chimeric human and murine TCR beta polypeptide.

According to some embodiments of the invention, the TCR alpha polypeptide comprises an amino acid sequence selected form the group consisting of SEQ ID NO: 8, 14, 17 and 20-23.

According to some embodiments of the invention, the TCR alpha polypeptide comprises an amino acid sequence selected form the group consisting of SEQ ID NO: 14, 17 and 20-23.

According to some embodiments of the invention, the TCR beta polypeptide comprises an amino acid sequence selected form the group consisting of SEQ ID NO: 10-11, 15-16, 18-19 and 24-25.

According to some embodiments of the invention, the TCR beta polypeptide comprises an amino acid sequence selected form the group consisting of SEQ ID NO: 15-16, 18-19 and 24-25.

According to an aspect of some embodiments of the present invention there is provided at least one polynucleotide encoding the TCR.

According to an aspect of some embodiments of the present invention there is provided a transduced T cell expressing the TCR complex or TCR or the at least one polynucleotide.

According to an aspect of some embodiments of the present invention there is provided a method of expressing a TCR in a T cell, the method comprising introducing into a T cell the at least one polynucleotide, under conditions which allow expression of the TCR.

According to some embodiments of the invention, the introducing is effected in-vitro or ex-vivo.

According to some embodiments of the invention, the T cell does not express an endogenous TCR.

According to some embodiments of the invention, the method further comprising downregulating expression of the endogenous TCR prior to the introducing.

According to an aspect of some embodiments of the present invention there is provided a method of treating a disease associated with a pathological cell in a subject in need thereof, the method comprising administering to the subject a therapeutically effective amount of the T cell; and a therapeutic composition capable of binding the pathological cell and the TCR complex, thereby treating the disease in the subject.

According to an aspect of some embodiments of the present invention there is provided the T cell; and a therapeutic composition capable of binding a pathological cell and the TCR complex, for use in treating a disease associated with the pathological cell in a subject in need thereof.

According to some embodiments of the invention, the T cell is allogeneic to the subj ect.

According to an aspect of some embodiments of the present invention there is provided an article of manufacture comprising a packaging material packaging the T cell; and a therapeutic composition capable of binding a pathological cell and the TCR complex.

According to some embodiments of the invention, the therapeutic composition comprises an anti-CD3 antibody.

According to some embodiments of the invention, the anti-CD3 antibody is selected from the group consisting of diL2K, TR66 and OKT3.

According to some embodiments of the invention, the pathological cell expresses CD19 and the therapeutic composition comprises Blinatumomab.

According to some embodiments of the invention, the pathological cell expresses EpCAM and the therapeutic composition comprises MT110.

According to some embodiments of the invention, the pathological cell is a cancerous cell.

According to some embodiments of the invention, the cancer is selected from the group consisting of lymphoma, leukemia, glioblastoma, colon cancer, gastric cancer, pancreatic cancer, ovarian cancer, lung cancer and skin cancer.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

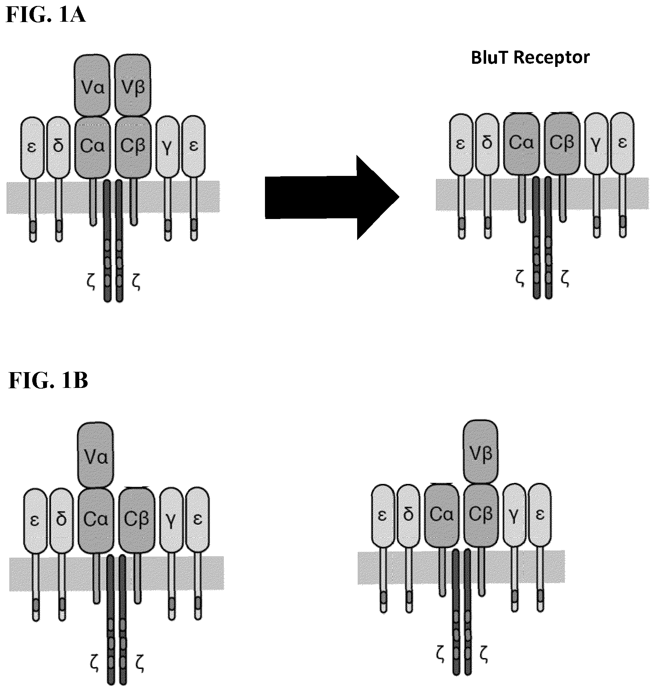

FIGs. 1A-B shows schematic representations of the engineered TCR complex of some embodiments of the invention. Figure 1A demonstrates a TCR complex comprising TCR alpha and beta chains that are devoid of the variable alpha and beta regions. This TCR is referred to herein as "blunt truncated TCR (BluT)". Figure 1B demonstrates a TCR complex comprising mismatch pairing of exogenous TCR alpha and beta chains devoid of variable regions with endogenous TCR alpha and beta chains.

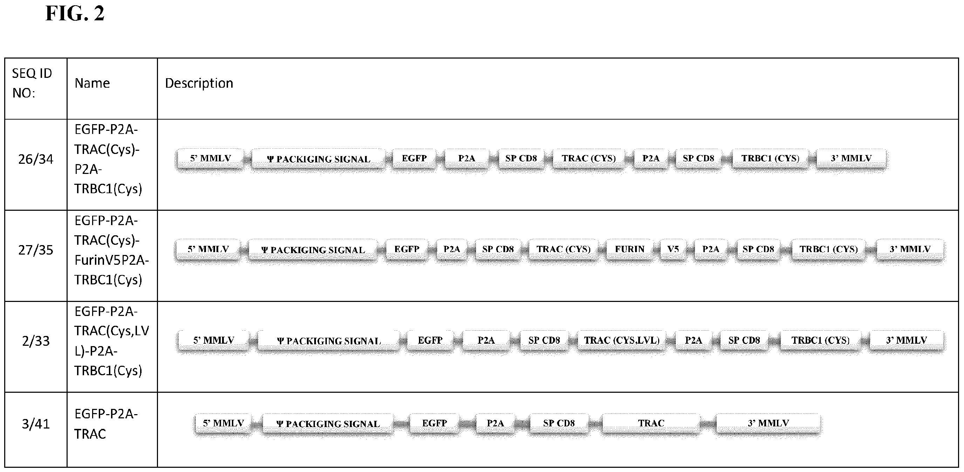

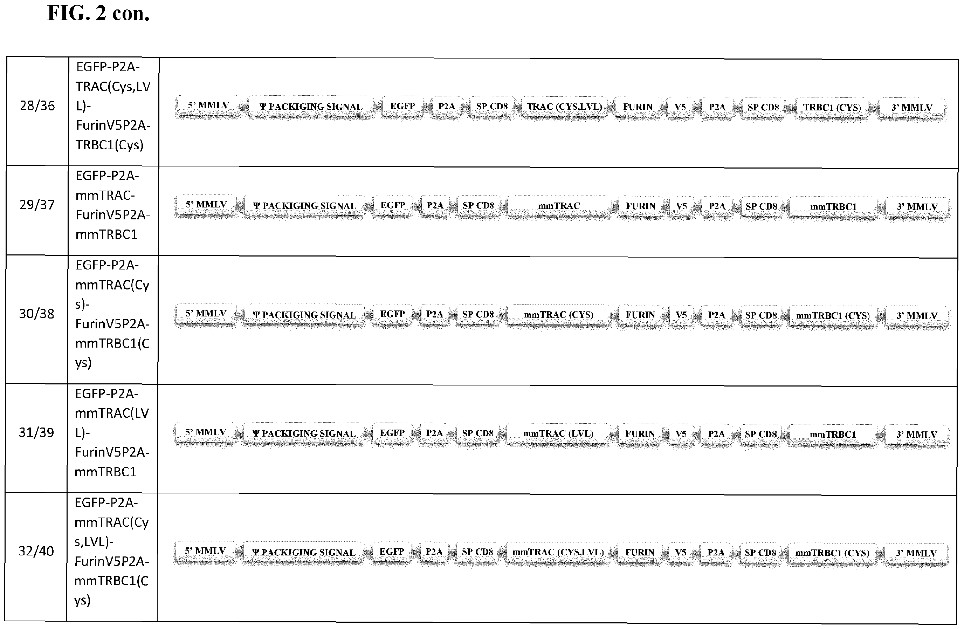

FIG. 2 shows schematic representations of the engineered TCR of some embodiments of the invention. When indicated, 3 mutations were introduced in the transmembrane domain of the alpha constant chain: S116L, G119V and F120L (marked as LVL). When indicated, cysteines were introduced in both the alpha and beta chains (marked as CYS): T48C in the alpha chain and S57C in the beta chain. When indicated, several mutations were introduced in the extracellular domain of the constant alpha and beta domains, as follows: constant alpha mutations P90S, E91D, S92V, S93P; constant beta mutations: E18K, S22A, F133I, E/V136A, Q139H (marked as mm).

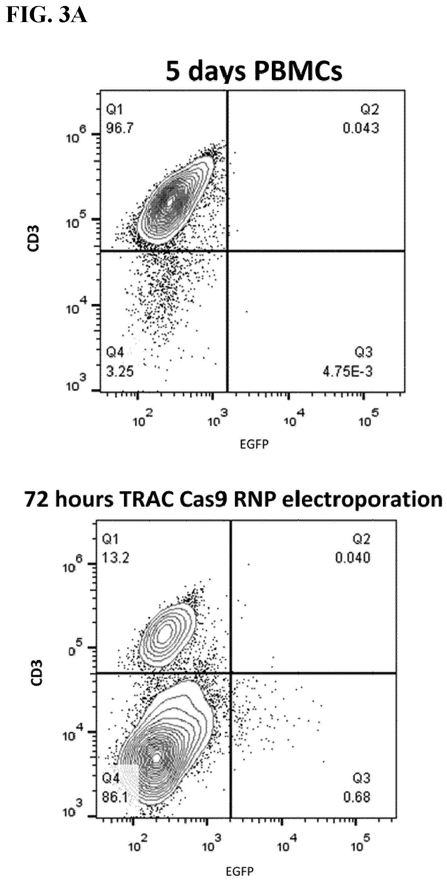

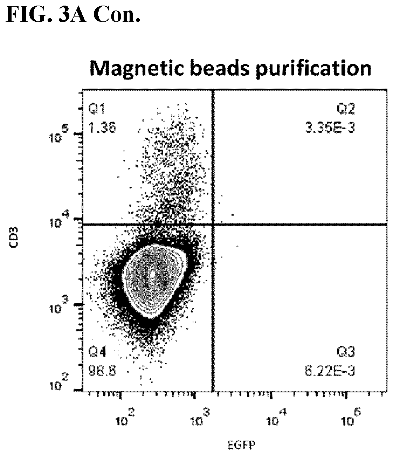

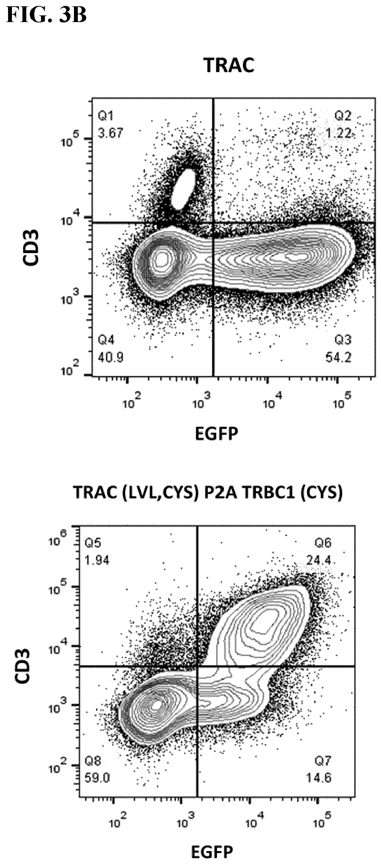

FIGs. 3A-B demonstrates re-expression of CD3 in TCR negative T cells following expression of BluT. Figure 3A shows generation of CD3-/TCR- T cells by electroporation with Cas9 RNP and gRNA targeting the TCR alpha chain (SEQ ID NO: 1) followed by magnetic beads purification of the CD3-/TCR- T cells. Figure 3B shows re-expression of CD3 in the T cells shown in Figure 3A following infection with a construct encoding a truncated alpha chain comprising an additional cysteine and several transmembrane hydrophobic mutations and a truncated beta chain comprising an additional cysteine, (TRAC(Cys,LVL)-P2A-TRBC1(Cys), SEQ ID NO: 2 as compared to no expression following infection with a construct encoding only a truncated alpha chain (TRAC, SEQ ID NO: 3), as determined by flow cytometry using an anti-CD3 OKT3 antibody. EGFP serves as a marker of infection.

### DESCRIPTION OF SPECIFIC EMBODIMENTS OF THE INVENTION

The present invention, in some embodiments thereof, relates to engineered TCR and methods of using same.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Cancer immunotherapy has emerged in the last couple of years as a promising strategy for treating various types of cancer. Cell-based therapy using e.g. T cells having an engineered T cell receptor (TCR) or CAR T cells, antibody-based cancer immunotherapies, and combinations thereof were shown to exert anti-tumor effects in several types of cancers.

Whilst reducing specific embodiments of the present invention to practice, the present inventors designed and expressed a truncated TCR that is devoid of the variable regions of the TCR alpha and beta chains which presented as part of a TCR complex on the surface of T cells devoid of an endogenous TCR (Example 1 of the Examples section which follows).

Consequently, specific embodiments of the present teachings suggest T cells genetically engineered to express the truncated TCR in combination with a therapeutic composition capable of binding a pathological cell on the one hand and the TCR complex on the other hand (e.g. a T cell engager antibody) to treat diseases associated with pathologic cells (e.g. cancer).

Thus, according to an aspect of the present invention, there is provided a T cell receptor (TCR) complex comprising a TCR comprising a TCR alpha polypeptide and a TCR beta polypeptide, wherein said TCR is devoid of an antigen-binding domain, and a CD3 polypeptide devoid of a heterologous antigen-binding domain; and wherein said TCR complex is capable of being presented on a surface of a T cell expressing same.

According to an additional or an alternative aspect of the present invention, there is provided a T cell receptor (TCR) comprising a human TCR alpha polypeptide and a human TCR beta polypeptide, wherein said TCR is devoid of an antigen-binding domain, and wherein said TCR alpha and beta polypeptides comprise amino acid modifications enabling presentation of said TCR as a TCR complex on a surface of a T cell expressing same.

As used herein, the term "T cell receptor (TCR)" refers to a receptor comprising a hetero-dimer of a TCR alpha polypeptide and a TCR beta polypeptide, which is capable of being presented (or presentable) as a TCR complex on a surface of a T cell expressing same.

As used herein, the term "TCR complex" refers to a complex formed by the association of CD3 with a TCR.

As used herein, the term "CD3" refers to the polypeptide expression product of the *CD3G, CD3D, CD3E* or *CD247* gene (Gene ID 917, 915, 916, 919, respectively), and includes CD3γ, CD3δ, CD3ε and CD3zeta.According to specific embodiments, CD3 is human CD3.

According to a specific embodiment, the CD3 is endogenous to the cell it is expressed in.

According to a specific embodiment, the CD3 refers to the human CD3γ polypeptide, such as provided in the following Accession No. NP_000064 or SEQ ID NO: 4.

According to a specific embodiment, the CD3 refers to the human CD3δ, such as provided in the following Accession Nos. NP_000723, NP_001035741 or SEQ ID NO: 5.

According to a specific embodiment, the CD3 refers to the human CD3ε, such as provided in the following Accession No. NP_000724 or SEQ ID NO: 6.

According to a specific embodiment, the CD3zeta (CD247) refers to the human, such as provided in the following Accession No. NP_000725, NP_932170, NP_001365444, NP_001365445 or SEQ ID NO: 7.

Herein, the "CD3 polypeptide" or "CD3 chain" refers to full length CD3 or a fragment or a homolog thereof, which comprises a signaling domain and maintains at least the capability of CD3 of being presented as a TCR complex on a surface of a T cell expressing same. Such homologues can be, for example, at least 70 %, at least 75 %, at least 80 %, at least 81 %, at least 82 %, at least 83 %, at least 84 %, at least 85 %, at least 86 %, at least 87 %, at least 88 %, at least 89 %, at least 90 %, at least 91 %, at least 92 %, at least 93 %, at least 94 %, at least 95 %, at least 96 %, at least 97 %, at least 98 %, at least 99 % or 100 % identical or homologous to the polypeptide set forth in SEQ ID NO: 4-7.

Thus, for example, a TCR complex can be composed of a CD3γ chain, a CD3δ chain, two CD3ε chains, a homodimer of CD3zeta chains, a TCR alpha polypeptide, and a TCR beta polypeptide.

Methods of determining presentation of a TCR complex are well known in the art and include for example Flow cytometry or immunostaining using an anti-CD3 antibody.

The TCR disclosed herein is devoid of an antigen-binding domain.

According to specific embodiments, the CD3 polypeptide is devoid of a heterologous antigen-binding domain.

According to specific embodiments, wherein the TCR comprises a human TCR alpha and a human TCR beta polypeptides comprising the amino acid modifications enabling presentation of the TCR as a TCR complex on a surface of a T cell expressing same, the CD3 polypeptide may comprise a heterologous antigen-binding domain.

As used herein, the term "heterologous" refers to an amino acid sequence or residue which is not native to the recited amino acid sequence (e.g. TCR alpha polypeptide, a TCR beta polypeptide, a CD3 polypeptide) at least in localization or is completely absent from the native sequence of the recited amino acid sequence.

Hence, according to specific embodiments, all the components of the TCR complex are devoid of an antigen-binding domain.

As used herein, the phrase "antigen-binding domain" refers to the domain that contains an amino acid sequence which confers binding to an antigen in a specific manner i.e., Kd of 10⁻⁴M or lower, of a TCR or an antibody. Typically, such a domain in the context of the present invention includes the variable domains of the TCR alpha and beta chains of a TCR or the variable domains or the heavy and light chains of an antibody.

Thus, the TCR is truncated of a naturally occurring antigen-binding domain i.e., is devoid of a naturally occurring antigen-binding domain of the naturally occurring TCR.
In other words, the TCR alpha and beta polypeptides; and the CD3 polypeptide of some embodiments of the invention, are not translationally fused to an antigen-binding domain

The TCR alpha and beta polypeptides or CD3 polypeptide of some embodiments may be attached to an antigen-binding domain not as a translational fusion (e.g. an antibody comprising an antigen-binding domain having a binding specificity to the TCR complex).

According to other specific embodiments, the TCR alpha and/or beta polypeptides are not attached to a non-translationally fused antigen-binding domain.

According to specific embodiments, the TCR and/or CD3 polypeptide is devoid of a heterologous extracellular binding domain capable of binding a target that is presented on a cell surface of a target cell of a T cell. In other words, the TCR alpha and beta polypeptides and/or CD3 polypeptide are not translationally fused to a heterologous extracellular binding domain capable of binding a target.

As used herein, the phrase "extracellular binding domain capable of binding a target" refers to a proteinaceous moiety having a binding affinity i.e., Kd of 10⁻⁴M or lower, to a target of interest being presented on a target cell. Non-limiting examples of binding domains include the binding domain of a receptor, the binding domain of a ligand, the binding domain of a hormone (e.g. leptin) and an antigen-binding domain, as further described hereinabove.

Assays for testing binding are well known in the art and include, but not limited to flow cytometry, immunostaining, bio-layer interferometry Blitz^{®} assay, HPLC, surface plasmon resonance (e.g. Biacore).

Herein, the "TCR alpha polypeptide" refers to a fragment of a TCR alpha chain or a homolog thereof, comprising an extracellular domain devoid of an antigen-binding domain (i.e., devoid of a variable domain), a transmembrane domain, and optionally an intracellular domain, which maintains at least the capability of a TCR alpha chain of being presented as a TCR complex on a surface of a T cell expressing same.

As used herein, "TCR alpha" or "TCR alpha chain" refers to the polypeptide expression product of the *TRA* gene (Corresponding to human Gene ID 6955). According to specific embodiments, TCR alpha refers to the human TCR alpha. According to specific embodiments, TCR alpha refers to the mouse TCR alpha.

According to specific embodiments, the TCR alpha polypeptide comprises an amino acid sequence of a TCR alpha constant domain or a fragment or homolog thereof, a TCR alpha hinge region, a TCR alpha transmembrane domain, and a TCR alpha intracellular domain.

According to specific embodiments, the TCR alpha polypeptide is less than 170, less than 160, less than 155, or less than 152 amino acids long, each possibility represents a separate embodiment of the present invention.

According to specific embodiments, the TCR alpha polypeptide is at least 120, at least 125, at least 130 at least 140 or at least 150 amino acids long, each possibility represents a separate embodiment of the present invention.

According to specific embodiments, the TCR alpha polypeptide is 140 - 151 amino acids long.

According to specific embodiments, the TCR alpha polypeptide is about 141 amino acids long.

According to specific embodiments, the TCR alpha polypeptide is about 151 amino acids long.

According to specific embodiments, the TCR alpha polypeptide is about 145 amino acids long.

According to specific embodiments, the TCR alpha polypeptide is about 150 amino acids long.

According to specific embodiments, the TCR alpha polypeptide comprises a murine TCR alpha polypeptide.

A non-limiting example of a murine TCR alpha polypeptide that can be used with specific embodiments of the invention is provided in Uniprot ID A0A075B662 (SEQ ID NO: 8).

According to specific embodiments, the TCR alpha polypeptide comprises a human TCR alpha polypeptide.

A non-limiting example of a human TCR alpha polypeptide devoid of a variable domain is provided in Uniprot ID P01848 (SEQ ID NO: 9).

Herein the "TCR beta polypeptide" refers to a fragment of a TCR beta chain or a homolog thereof, comprising an extracellular domain devoid of an antigen-binding domain (i.e., devoid of a variable domain), a transmembrane domain, and optionally an intracellular domain, which maintain at least the capability of a TCR beta chain of being presented as a TCR complex on a surface of a T cell expressing same.

As used herein, "TCR beta" or "TCR beta chain" refers to the polypeptide expression product of the *TRB* gene (Corresponding to human Gene ID 6957) and includes TRBC1 (Corresponding to the human Gee ID 28639) and TRBC2 (Corresponding to human Gene ID 28638). According to specific embodiments, TCR beta refers to TRBC1. According to specific embodiments, TCR beta refers to TRBC2. According to specific embodiments, TCR beta refers to the human TCR beta. According to specific embodiments, TCR beta refers to the mouse TCR beta.

According to specific embodiments, the TCR beta polypeptide comprises an amino acid sequence of a TCR beta constant domain or a fragment or homolog thereof, a TCR beta hinge region, a TCR beta transmembrane domain, and a TCR beta intracellular domain.

According to specific embodiments, the TCR beta polypeptide is less than 250, less than 200, less than 190, or less than 188 amino acids long, each possibility represents a separate embodiment of the present invention.

According to specific embodiments, the TCR beta polypeptide is at least 140, at least 150, at least 160, at least 170, at least 180 or at least 185 amino acids long, each possibility represents a separate embodiment of the present invention.

According to specific embodiments, the TCR beta polypeptide is 170-187 amino acids long.

According to specific embodiments, the TCR beta polypeptide is about 172 amino acids long.

According to specific embodiments, the TCR beta polypeptide is about 178 amino acids long.

According to specific embodiments, the TCR beta polypeptide is about 183 amino acids long. According to specific embodiments, the TCR beta polypeptide is about 187 amino acids long.

According to specific embodiments, the TCR beta polypeptide comprises a murine TCR beta polypeptide.

Non-limiting examples of murine TCR beta polypeptides that can be used with specific embodiments of the invention are provided in SEQ ID NOs: 10-11.

According to specific embodiments, the TCR beta polypeptide comprise a human TCR beta polypeptide.

Non-limiting examples of human TCR beta polypeptides devoid of a variable domain are provided in SEQ ID NOs: 12-13.

In order to improve presentation of a TCR having a human TCR alpha and beta polypeptides devoid of an antigen-binding domain the present inventors have envisaged modifications in the sequences of the human TCR alpha and beta polypeptides. Non-limiting examples of such modifications include insertion of a heterologous dimerizing moiety and insertion of a minimal murine amino acid sequence.

Thus, according to specific embodiments, the TCR alpha polypeptide and/or TCR beta polypeptide comprise a chimeric human and murine TCR alpha polypeptide and/or TCR beta polypeptide. Thus, for Example the TCR alpha and/or beta polypeptide may be based on human TCR alpha and/or beta comprising a short (e.g. 3 - 20) murine amino acid sequence. Such sequences are known in the art and disclosed in e.g. Sommermeyer D, et al. J Immunol. 2010, the contents of which are fully incorporated herein by reference. Thus, according to specific embodiments, the TCR alpha polypeptide comprises a human TCR alpha polypeptide comprising the amino acid changes P90S, E91D, S92V and S93P corresponding to TCR alpha amino acid sequence as set forth in SEQ ID NO: 9. According to specific embodiments, the TCR beta polypeptide comprises a human TCR beta polypeptide comprising the amino acid changes E18K, S22A, F133I, E/V136A, Q139H corresponding to TCR beta amino acid sequence as set forth in SEQ ID NO: 12.

As used herein, the phrase "corresponding to SEQ ID NO: 9" intends to include the corresponding amino acid residue relative to any other TCR alpha amino acid sequence.

As used herein, the phrase "corresponding to SEQ ID NO: 12" intends to include the corresponding amino acid residue relative to any other TCR beta amino acid sequence.

Thus, according to specific embodiments, the TCR alpha polypeptide comprises SEQ ID NO: 14 and the TCR beta polypeptide comprises SEQ ID NO: 15 or 16.

According to specific embodiments, the TCR alpha and/or beta polypeptide may be based on human TCR alpha and/or beta comprising the hinge region of mouse TCR alpha and/or beta, respectively.

According to specific embodiments, the TCR alpha and/or beta polypeptide may comprise the transmembrane domain and optionally the intracellular domain of human TCR alpha and/or beta, respectively, and the extracellular domain of mouse TCR alpha and/or beta, respectively.

According to specific embodiments, the TCR comprises a heterologous dimerizing moiety.

According to specific embodiments, the heterologous dimerizing moiety is located in the extracellular domain of the TCR.

According to specific embodiments, the heterologous dimerizing moiety is located in the constant domain of the TCR.

According to specific embodiments, the heterologous dimerizing moiety is located in the hinge domain of the TCR.

As used herein, the term "dimerizing moiety" refers to a heterologous amino acid sequence capable of forming a TCR alpha polypeptide - TCR beta polypeptide hetero-dimer. Such an amino acid may include for example an amino acid sequence comprising at least two cysteine residues, one in the TCR alpha polypeptide and the second in the TCR beta polypeptide, enabling the formation of a disulfide bond between the thiol groups. Another example is dimerization domain or an amino acid sequence enabling steric formation of a dimer. Such sequences are known to the skilled in the art. Methods of determining dimerization are known in the art, including but not limited to immunoprecipitation, size exclusion chromatography, fast protein liquid chromatography (FPLC), multi-angle light scattering (SEC-MALS) analysis, SDS-PAGE analysis, nano-DSF, yeast two-hybrid system (e.g. RRS) and flow cytometry.

Any known dimerizing moiety known in the art can be used with specific embodiments of the invention. Non-limiting examples of dimerizing moieties that can be used with specific embodiments of the invention include a heterologous cysteine residue at each of the TCR alpha and TCR beta polypeptides, an Fc domain of an antibody (not comprising the antibody antigen-binding domain), murine amino acid sequences capable of forming a heterodimer such as the short murine amino acid sequences described hereinabove, FRB-FKBP, leucine zipper.

According to specific embodiments, the TCR alpha and beta polypeptides each comprise at least one heterologous cysteine.

According to specific embodiments, the heterologous cysteine is located in the extracellular domain of each of the TCR alpha and beta polypeptides.

According to specific embodiments, the heterologous cysteine is located in the hinge or constant domain of each of the TCR alpha and beta polypeptides.

According to a specific embodiment, the TCR alpha polypeptide comprises a T48C amino acid substitution corresponding to the TCR alpha amino acid sequence as set forth in SEQ ID NO: 9 and the TCR beta polypeptide comprises a S57C amino acid substitution corresponding to the TCR beta amino acid sequence as set forth in SEQ ID NO: 12.

Thus, according to specific embodiments, the TCR alpha polypeptide comprises SEQ ID NO: 17 and the TCR beta polypeptide comprises SEQ ID NO: 18 or 19.

As each of the TCR alpha and beta polypeptides comprise an endogenous cysteine located in the hinge regions of the polypeptides forming a single disulfide bond between the alpha and beta polypeptides, the addition of at least one heterologous cysteine results in the presence of at least two cysteine residues in each of the TCR alpha and beta polypeptides enabling the formation of at least two disulfide bonds between the TCR alpha and TCR beta polypeptides.

Thus, according to specific embodiments, the TCR alpha and beta polypeptides each comprise at least two cysteine residues capable of forming at least two disulfide bonds between the alpha and beta polypeptides.

According to specific embodiments, the at least two cysteine residues are located in the extracellular domain of each of the TCR alpha and beta polypeptides.

According to specific embodiments, at least one cysteine residue is located in the hinge region of each of the TCR alpha and beta polypeptides and at least one cysteine residue is located in the hinge or constant domain of each of the TCR alpha and beta polypeptides.

The terms "TCR alpha polypeptide" and "TCR beta polypeptide" also encompass functional homologues (naturally occurring or synthetically/recombinantly produced), which exhibit the desired activity (*i.e.,* being presented as a TCR complex on a surface of a T cell expressing same). Such homologues can be, for example, at least 70 %, at least 75 %, at least 80 %, at least 81 %, at least 82 %, at least 83 %, at least 84 %, at least 85 %, at least 86 %, at least 87 %, at least 88 %, at least 89 %, at least 90 %, at least 91 %, at least 92 %, at least 93 %, at least 94 %, at least 95 %, at least 96 %, at least 97 %, at least 98 %, at least 99 % or 100 % identical or homologous to the polypeptide set forth in SEQ ID NO: 8, 9, 14 or 17 for TCR alpha and SEQ ID NO: 10-13, 15-16 or 18-19 for TCR beta (as further described hereinbelow).

Sequence identity or homology can be determined using any protein or nucleic acid sequence alignment algorithm such as Blast, ClustalW, and MUSCLE.

The homolog may also refer to an ortholog, a deletion, insertion, or substitution variant, including an amino acid substitution, as further described hereinbelow.

According to specific embodiments, the TCR alpha polypeptide and/or TCR beta polypeptide may comprise conservative and/or non-conservative amino acid substitutions. Non-limiting examples of such substitution are known in the art and disclosed in e.g. Haga-Friedman A, et al. J Immunol. 2012; 188:5538-46; Froning K, et al. Nat Commun [Internet]. Springer US; 2020; 11:1-14; Boulter JM, et al. Protein Eng. 2003; 16:707-11, the contents of which are fully incorporated herein by reference.

According to specific embodiments, the TCR alpha polypeptide and/or TCR beta polypeptide do not comprise conservative and/or non-conservative amino acid substitutions in an endogenous cysteine residue located in the hinge region of each of the TCR alpha and beta.

The term "conservative substitution" as used herein, refers to the replacement of an amino acid present in the native sequence in the peptide with a naturally or non-naturally occurring amino or a peptidomimetics having similar steric properties. Where the side-chain of the native amino acid to be replaced is either polar or hydrophobic, the conservative substitution should be with a naturally occurring amino acid, a non-naturally occurring amino acid or with a peptidomimetic moiety which is also polar or hydrophobic (in addition to having the same steric properties as the side-chain of the replaced amino acid).

As naturally occurring amino acids are typically grouped according to their properties, conservative substitutions by naturally occurring amino acids can be easily determined bearing in mind the fact that in accordance with the invention replacement of charged amino acids by sterically similar non-charged amino acids are considered as conservative substitutions.

For producing conservative substitutions by non-naturally occurring amino acids it is also possible to use amino acid analogs (synthetic amino acids) well known in the art. A peptidomimetic of the naturally occurring amino acid is well documented in the literature known to the skilled practitioner.

When affecting conservative substitutions the substituting amino acid should have the same or a similar functional group in the side chain as the original amino acid.

The phrase "non-conservative substitutions" as used herein refers to replacement of the amino acid as present in the parent sequence by another naturally or non-naturally occurring amino acid, having different electrochemical and/or steric properties. Thus, the side chain of the substituting amino acid can be significantly larger (or smaller) than the side chain of the native amino acid being substituted and/or can have functional groups with significantly different electronic properties than the amino acid being substituted. Examples of non-conservative substitutions of this type include the substitution of phenylalanine or cycohexylmethyl glycine for alanine, isoleucine for glycine, or -NH-CH[(-CH₂)₅-COOH]-CO- for aspartic acid. Those non-conservative substitutions which fall under the scope of the present invention are those which still constitute a peptide having neuroprotective properties.

Thus, according to specific embodiments, one or more amino acid changes in the TCR alpha are selected from the group consisting of S116L, G119V and F120L corresponding to the TCR alpha amino acid sequence as set forth in SEQ ID NO: 9.

According to specific embodiments, one or more amino acid changes in the TCR alpha are selected from the group consisting of S21F, T32I, A72T corresponding to the TCR alpha amino acid sequence as set forth in SEQ ID NO: 9.

According to other specific embodiments, the TCR alpha polypeptide does not comprise one or more amino acid changes selected from the group consisting of S21F, T32I, A72T corresponding to the TCR alpha amino acid sequence as set forth in SEQ ID NO: 9.

According to specific embodiments, one or more amino acid changes in the TCR beta are selected from the group consisting of E18K, H23R, D39P, S54D corresponding to the TCR beta amino acid sequence as set forth in SEQ ID NO: 12.

According to other specific embodiments, the TCR beta polypeptide does not comprise one or more amino acid changes selected from the group consisting of E18K, H23R, D39P, S54D corresponding to the TCR beta amino acid sequence as set forth in SEQ ID NO: 12.

According to specific embodiments, the TCR alpha polypeptide comprises an amino acid sequence having at least 70 %, at least 80 % at least 85 %, at least 90 %, at least 95 %, at least 96 %, at least 97 %, at least 98 %, at least 99 % or 100 % identity to an amino acid sequence selected form the group consisting of SEQ ID NO: 8, 14, 17 and 20-23, each possibility represents a separate embodiment of the present invention.

According to specific embodiments, the TCR alpha polypeptide comprises an amino acid sequence selected form the group consisting of SEQ ID NO: 8, 14, 17 and 20-23.

According to specific embodiments, the TCR alpha polypeptide comprises an amino acid sequence having at least 70 %, at least 80 % at least 85 %, at least 90 %, at least 95 %, at least 96 %, at least 97 %, at least 98 %, at least 99 % or 100 % identity to an amino acid sequence selected form the group consisting of SEQ ID NO: 14, 17 and 20-23, each possibility represents a separate embodiment of the present invention.

According to specific embodiments, the TCR alpha polypeptide comprises an amino acid sequence selected form the group consisting of SEQ ID NO: 14, 17 and 20-23.

According to specific embodiments, the TCR beta polypeptide comprises an amino acid sequence having at least 70 %, at least 80 % at least 85 %, at least 90 %, at least 95 %, at least 96 %, at least 97 %, at least 98 %, at least 99 % or 100 % identity to an amino acid selected form the group consisting of SEQ ID NO: 10-11, 15-16, 18-19 and 24-25, each possibility represents a separate embodiment of the present invention.

According to specific embodiments, the TCR beta polypeptide comprises an amino acid sequence selected form the group consisting of SEQ ID NO: 10-11, 15-16, 18-19 and 24-25.

According to specific embodiments, the TCR beta polypeptide comprises an amino acid sequence having at least 70 %, at least 80 % at least 85 %, at least 90 %, at least 95 %, at least 96 %, at least 97 %, at least 98 %, at least 99 % or 100 % identity to an amino acid selected form the group consisting of SEQ ID NO: 15-16, 18-19 and 24-25, each possibility represents a separate embodiment of the present invention.

According to specific embodiments, the TCR beta polypeptide comprises an amino acid sequence selected form the group consisting of SEQ ID NO: 15-16, 18-19 and 24-25.

According to specific embodiments, the TCR alpha polypeptide comprises SEQ ID NO: 8 and the TCR beta polypeptide comprises SEQ ID NO: 10 or 11.

According to specific embodiments, the TCR alpha polypeptide comprises SEQ ID NO: 14 and the TCR beta polypeptide comprises SEQ ID NO: 15 or 16.

According to specific embodiments, the TCR alpha polypeptide comprises SEQ ID NO: 17 or 20 and the TCR beta polypeptide comprises SEQ ID NO: 18 or 19.

According to specific embodiments, the TCR alpha polypeptide comprises SEQ ID NO: 21 or 23 and the TCR beta polypeptide comprises SEQ ID NO: 24 or 25.

According to specific embodiments, the TCR alpha polypeptide comprises SEQ ID NO: 22 and the TCR beta polypeptide comprises SEQ ID NO: 15 or 16.

According to specific embodiments, the TCR may comprise a heterologous amino acid sequence translationally fused to the TCR alpha and/or beta polypeptides, as long as it is not an antigen-binding domain. Non-limiting examples of such heterologous sequences may comprise an amino acid sequence of a trafficking sequence (e.g. a CD8 membrane trafficking peptide such as provided in SEQ ID NO: 42), a receptor, a ligand or a hormone, a signaling domain.

According to specific embodiments, the TCR comprises a heterologous domain of a receptor or a ligand. Non-limiting examples of such receptors include EGFR, HER2, VEGFR1, VEGFR2, EpoR, FGFR1, FGFR2, FGFR3, FGFR4, MPL, CSF3R, CSF2RA, FLT3, CD117, PD1, CTLA4, CD28, Designed Ankyrin Repeat Protein (DARPin). Non-limiting examples of such ligands include TPO, IL2, IL15, IL18, SCF.

According to specific embodiments, the TCR is devoid of a heterologous domain of a receptor or a ligand.

According to specific embodiments, the TCR comprises a heterologous signaling domain, which may be an activating or inhibitory signaling domain.

As used herein, the phrase "activating signal" refers to an amino acid sequence capable of transmitting a primary stimulatory signal or a co-stimulatory signal resulting in T cell proliferation, maturation, cytokine production and/or induction of regulatory or effector functions. Typically, an activating primary stimulatory signal domain comprises an ITAM domain and a co-stimulatory signal domain does not comprise an ITAM domain.

Any known activating signal domain can be used with specific embodiments of the present invention. Non-limiting examples of activating signaling domains include the intracellular signaling domain of the proteins CD3zeta, FcR gamma, FcR beta, CD5, CD22, CD79a, CD79b, CD66d, 4-1BB, CD28, OX40, ICOS, CD27, ICOS, GITR, HVEM, TIM1, LFA1(CD11a), CD2, CD40L, LIGHT, CD30, Fc receptor, DAP10 and DAP12.

As used herein the phrase "inhibitory signal" refers to an amino acid sequence capable of transmitting a primary or a secondary inhibitory signal resulting in suppression of T cell proliferation, maturation, cytokine production and/or induction of regulatory or effector functions. According to specific embodiments, the inhibitory signal domain is an intracellular domain comprising an ITIM domain.

Any known inhibitory signal domain can be used with specific embodiments of the present invention. Non-limiting examples of inhibitory signaling domains include the intracellular signaling domains of the proteins PD1, CTLA4, LAG3, TIM3, BTLA, TIGIT, 2B4, CD300LF, PECAM, LY9, SIRPA and CD244.

Methods of determining signaling of an activating or inhibitory signal in T cells are well known in the art and include, but are not limited to, binding assay using e.g. BiaCore, HPLC or flow cytometry, enzymatic activity assays such as kinase activity assays, and expression of molecules involved in the signaling cascade using e.g. PCR, Western blot, immunoprecipitation and immunohistochemistry. Additionally, or alternatively, determining transmission of a signal can be effected by evaluating T cell activation or function. Methods of evaluating T cell activation or function are well known in the art and include, but are not limited to, proliferation assays such as BRDU and thymidine incorporation, cytotoxicity assays such as chromium release, cytokine secretion assays such as intracellular cytokine staining ELISPOT and ELISA, expression of activation markers such as CD25, CD69 and CD69 using flow cytometry.

According to other specific embodiments, the TCR is devoid of a heterologous signaling domain.

According to specific embodiments, the TCR comprises a heterologous tag or is bound to a tag.

According to specific embodiments, the tag is a detectable moiety.

Examples of detectable moieties that can be used in the present invention include but are not limited to radioactive isotopes (such as ^{[125]}iodine), phosphorescent chemiluminescent or fluorescent chemicals and enzymes [e.g. horseradish peroxidase (HPR), beta-galactosidase and alkaline phosphatase (AP)]. Further examples of detectable moieties include those detectable by Positron Emission Tomagraphy (PET) and Magnetic Resonance Imaging (MRI), all of which are well known to those of skill in the art.

Non-limiting examples of tags that can be used with specific embodiments include chitin binding protein (CBP)-tag, maltose binding protein (MBP)-tag, glutathione-S-transferase (GST)-tag, poly(His)-tag, FLAG tag, biotin, histidine, Epitope tags, such as, V5-tag, c-myc-tag, and HA-tag, and fluorescence tags such as green fluorescent protein (GFP or EGFP), red fluorescent protein (RFP), yellow fluorescent protein (YFP), blue fluorescent protein (BFP), and cyan fluorescent protein (CFP); as well as derivatives of these tags, or any tag known in the art.

Non-limiting schematic representations and sequences of the TCR of some embodiments of the invention are provided in Figure 2 and SEQ ID NOs: 2 and 26-32.

Typically, the TCR disclosed herein is produced by recombinant DNA technology.

Thus, according to an aspect of the present invention, there is provided at least one polynucleotide encoding the TCR or TCR complex.

As used herein the term "polynucleotide" refers to a single or double stranded nucleic acid sequence which is isolated and provided in the form of an RNA sequence, a complementary polynucleotide sequence (cDNA), a genomic polynucleotide sequence and/or a composite polynucleotide sequences (e.g., a combination of the above).

According to specific embodiments, the at least one polynucleotide comprises a nucleic acid sequence encoding the TCR alpha polypeptide; and a nucleic acid sequence encoding the TCR beta polypeptide.

According to specific embodiments, the TCR alpha and beta polypeptides are encoded by a single polynucleotide. Further description on expression of multiple polypeptides from a single polynucleotide is provided hereinbelow.

Thus, according to specific embodiments, the at least one polynucleotide is one polynucleotide.

According to other specific embodiments, distinct polynucleotides are used to encode the TCR alpha and beta polypeptides.

Thus, according to specific embodiments, the at least one polynucleotide is at least two polynucleotides.

According so specific embodiments, the at least one polynucleotide is two polynucleotides.

According to specific embodiments, the at least one polynucleotide further comprises a nucleic acid sequence encoding a CD3 polypeptide.

To express any of the disclosed polypeptides in a cell, a polynucleotide sequence encoding the polypeptide is preferably ligated into a nucleic acid construct suitable for cell expression. Such a nucleic acid construct includes at least one cisacting regulatory element for directing expression of the nucleic acid sequence. Cisacting regulatory sequences include those that direct constitutive expression of a nucleotide sequence as well as those that direct inducible expression of the nucleotide sequence only under certain conditions. Thus, for example, a promoter sequence for directing transcription of the polynucleotide sequence in the cell in a constitutive or inducible manner is included in the nucleic acid construct.

The nucleic acid construct (also referred to herein as an "expression vector") of some embodiments of the invention includes additional sequences which render this vector suitable for replication and integration (e.g., shuttle vectors). In addition, a typical cloning vectors may also contain a transcription and translation initiation sequence, transcription and translation terminator and a polyadenylation signal. By way of example, such constructs will typically include a 5' LTR, a tRNA binding site, a packaging signal, an origin of second-strand DNA synthesis, and a 3' LTR or a portion thereof.

The nucleic acid construct of some embodiments of the invention typically includes or encodes a signal sequence for targeting the polypeptide to the cell surface. According to a specific embodiment, the signal sequence for this purpose is a mammalian signal sequence or the signal sequence of the polypeptide variants of some embodiments of the invention.

Eukaryotic promoters typically contain two types of recognition sequences, the TATA box and upstream promoter elements. The TATA box, located 25-30 base pairs upstream of the transcription initiation site, is thought to be involved in directing RNA polymerase to begin RNA synthesis. The other upstream promoter elements determine the rate at which transcription is initiated.

Preferably, the promoter utilized by the nucleic acid construct of some embodiments of the invention is active in the specific cell population transformed, i.e. T cells. Examples of T cell specific promoters include lymphoid specific promoters [Calame et al., (1988) Adv. Immunol. 43:235-275]; in particular promoters of T-cell receptors [Winoto et al., (1989) EMBO J. 8:729-733].

Enhancer elements can stimulate transcription up to 1,000 fold from linked homologous or heterologous promoters. Enhancers are active when placed downstream or upstream from the transcription initiation site. Many enhancer elements derived from viruses have a broad host range and are active in a variety of tissues. For example, the SV40 early gene enhancer is suitable for many cell types. Other enhancer/promoter combinations that are suitable for some embodiments of the invention include those derived from polyoma virus, human or murine cytomegalovirus (CMV), the long term repeat from various retroviruses such as murine leukemia virus, murine or Rous sarcoma virus and HIV. See, Enhancers and Eukaryotic Expression, Cold Spring Harbor Press, Cold Spring Harbor, N.Y. 1983, which is incorporated herein by reference.

In the construction of the expression vector, the promoter is preferably positioned approximately the same distance from the heterologous transcription start site as it is from the transcription start site in its natural setting. As is known in the art, however, some variation in this distance can be accommodated without loss of promoter function.

Polyadenylation sequences can also be added to the expression vector in order to increase the efficiency of mRNA translation. Two distinct sequence elements are required for accurate and efficient polyadenylation: GU or U rich sequences located downstream from the polyadenylation site and a highly conserved sequence of six nucleotides, AAUAAA, located 11-30 nucleotides upstream. Termination and polyadenylation signals that are suitable for some embodiments of the invention include those derived from SV40.

In addition to the elements already described, the expression vector of some embodiments of the invention may typically contain other specialized elements intended to increase the level of expression of cloned nucleic acids or to facilitate the identification of cells that carry the recombinant DNA. For example, a number of animal viruses contain DNA sequences that promote the extra chromosomal replication of the viral genome in permissive cell types. Plasmids bearing these viral replicons are replicated episomally as long as the appropriate factors are provided by genes either carried on the plasmid or with the genome of the host cell.

The vector may or may not include a eukaryotic replicon. If a eukaryotic replicon is present, then the vector is amplifiable in eukaryotic cells using the appropriate selectable marker. If the vector does not comprise a eukaryotic replicon, no episomal amplification is possible. Instead, the recombinant DNA integrates into the genome of the engineered cell, where the promoter directs expression of the desired nucleic acid.

The expression vector of some embodiments of the invention can further include additional polynucleotide sequences that allow, for example, the translation of several proteins from a single mRNA such as an internal ribosome entry site (IRES) or a selfcleavable peptide e.g. a 2A peptide (e.g. P2A, T2A, E2A) which may be accompanied with a spacer and/or protease e.g. furin cleavage site (see e.g. Yang et al. Gene Ther. 2008; 15(21): 1411-1423); and sequences for genomic integration.

It will be appreciated that the individual elements comprised in the expression vector can be arranged in a variety of configurations. For example, enhancer elements, promoters and the like, and even the polynucleotide sequence(s) encoding the polypeptide can be arranged in a "head-to-tail" configuration, may be present as an inverted complement, or in a complementary configuration, as an anti-parallel strand. While such variety of configuration is more likely to occur with non-coding elements of the expression vector, alternative configurations of the coding sequence within the expression vector are also envisioned.

Examples for mammalian expression vectors include, but are not limited to, pcDNA3, pcDNA3.1(+/-), pGL3, pZeoSV2(+/-), pSecTag2, pDisplay, pEF/myc/cyto, pCMV/myc/cyto, pCR3.1, pSinRep5, DH26S, DHBB, pNMT1, pNMT41, pNMT81, which are available from Invitrogen, pCI which is available from Promega, pMbac, pPbac, pBK-RSV and pBK-CMV which are available from Strategene, pTRES which is available from Clontech, and their derivatives.

Expression vectors containing regulatory elements from eukaryotic viruses such as retroviruses can be also used. SV40 vectors include pSVT7 and pMT2. Vectors derived from bovine papilloma virus include pBV-1MTHA, and vectors derived from Epstein Bar virus include pHEBO, and p2O5. Other exemplary vectors include pMSG, pAV009/A⁺, pMTO10/A⁺, pMAMneo-5, baculovirus pDSVE, and any other vector allowing expression of proteins under the direction of the SV-40 early promoter, SV-40 later promoter, metallothionein promoter, murine mammary tumor virus promoter, Rous sarcoma virus promoter, polyhedrin promoter, or other promoters shown effective for expression in eukaryotic cells.

As described above, viruses are very specialized infectious agents that have evolved, in many cases, to elude host defense mechanisms. Typically, viruses infect and propagate in specific cell types. The targeting specificity of viral vectors utilizes its natural specificity to specifically target predetermined cell types and thereby introduce a recombinant gene into the infected cell. The ability to select suitable vectors for transforming T cells is well within the capabilities of the ordinary skilled artisan and as such no general description of selection consideration is provided herein.

Recombinant viral vectors are useful for *in vivo* expression of the polypeptides since they offer advantages such as lateral infection and targeting specificity. Lateral infection is inherent in the life cycle of, for example, retrovirus and is the process by which a single infected cell produces many progeny virions that bud off and infect neighboring cells. The result is that a large area becomes rapidly infected, most of which was not initially infected by the original viral particles. This is in contrast to vertical-type of infection in which the infectious agent spreads only through daughter progeny. Viral vectors can also be produced that are unable to spread laterally. This characteristic can be useful if the desired purpose is to introduce a specified gene into only a localized number of targeted cells.

Various methods can be used to introduce the expression vector of some embodiments of the invention into cells. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986] and include, for example, stable or transient transfection, lipofection, electroporation and infection with recombinant viral vectors. In addition, see U.S. Pat. Nos. 5,464,764 and 5,487,992 for positive-negative selection methods.

Introduction of nucleic acids by viral infection offers several advantages over other methods such as lipofection and electroporation, since higher transfection efficiency can be obtained due to the infectious nature of viruses.

Currently preferred *in vivo* nucleic acid transfer techniques include transfection with viral or non-viral constructs, such as adenovirus, lentivirus, Herpes simplex I virus, or adeno-associated virus (AAV) and lipid-based systems. Useful lipids for lipidmediated transfer of the gene are, for example, DOTMA, DOPE, and DC-Chol [Tonkinson et al., Cancer Investigation, 14(1): 54-65 (1996)]. The most preferred constructs for use in gene therapy are viruses, most preferably adenoviruses, AAV, lentiviruses, or retroviruses. A viral construct such as a retroviral construct includes at least one transcriptional promoter/enhancer or locus-defining element(s), or other elements that control gene expression by other means such as alternate splicing, nuclear RNA export, or post-translational modification of messenger. Such vector constructs also include a packaging signal, long terminal repeats (LTRs) or portions thereof, and positive and negative strand primer binding sites appropriate to the virus used, unless it is already present in the viral construct. In addition, such a construct typically includes a signal sequence for targeting the polypeptide to the desired site in a cell. According to specific embodiments, the signal sequence comprises a membrane trafficking sequence. Such sequences are known in the art. A non-limiting example of such a sequence is the CD8 signal peptide such as provided in SEQ ID No: 43. Optionally, the construct may also include a signal that directs polyadenylation, as well as one or more restriction sites and a translation termination sequence. By way of example, such constructs will typically include a 5' LTR, a tRNA binding site, a packaging signal, an origin of second-strand DNA synthesis, and a 3' LTR or a portion thereof. Other vectors can be used that are non-viral, such as cationic lipids, polylysine, and dendrimers.

According to specific embodiments, the polynucleotide is expressed in the cell while knocking out e.g. an endogenous TCR (i.e. knock-in / knock-out). Such methods are known in the art [see for example Menke D. Genesis (2013) 51: - 618; Capecchi, Science (1989) 244:1288-1292; Santiago et al. Proc Natl Acad Sci USA (2008) 105:5809-5814; International Patent Application Nos. WO 2014085593, WO 2009071334 and WO 2011146121; US Patent Nos. 8771945, 8586526, 6774279 and UP Patent Application Publication Nos. 20030232410, 20050026157, US20060014264; the contents of which are incorporated by reference in their entireties] and include targeted homologous recombination, site specific recombinases, PB transposases and genome editing by engineered nucleases (e.g. meganucleases, Zinc finger nucleases (ZFNs), transcription-activator like effector nucleases (TALENs) and CRISPR/Cas system).

Non-limiting schematic representations and nucleic acid constructs encoding the TCR of some embodiments of the invention are provided in Figure 2 and SEQ ID NOs: 33-40.

Specific embodiments of the present invention also contemplate T cells comprising the TCR or TCR complex described herein and method of generating same.

Thus, according to an aspect of the present invention, there is provided a transduced T cell expressing the TCR complex or the TCR or at least one polynucleotide encoding same.

According to an additional or an alternative aspect of the present invention, there is provided a method of expressing a TCR or a TCR complex in a T cell, the method comprising introducing into a T cell the at least one polynucleotide encoding the TCR disclosed herein, under conditions which allow expression of the TCR or the TCR complex.

Such conditions may be for example an appropriate temperature (e.g., 37 °C), atmosphere (e.g., air plus 5 % CO₂), pH, light, medium, supplements and the like.

According to other specific embodiments, the introducing is effected in-vivo.

According to specific embodiments, the introducing is effected in-vitro or ex-vivo.

As used herein, the term "T cell" refers to a differentiated lymphocyte expressing CD3, and includes CD4+ cells, CD8+ cells and NKT cells.

According to specific embodiments, the T cell is an effector cell.

As used herein, the term "effector T cell" refers to a T cell that activates or directs other immune cells e.g. by producing cytokines or has a cytotoxic activity e.g., CD4+, Th1/Th2, CD8+ cytotoxic T lymphocyte.

According to specific embodiments, the T cell is a regulatory cell.

As used herein, the term "regulatory T cell" or "Treg" refers to a T cell that negatively regulates the activation of other T cells, including effector T cells, as well as innate immune system cells. Treg cells are characterized by sustained suppression of effector T cell responses. According to a specific embodiment, the Treg is a CD4+CD25+Foxp3+ T cell.

According to specific embodiments, the T cell is a CD4+ T cell.

According to other specific embodiments, the T cell is a CD8+ T cell.

According to specific embodiments, the T cell is a naive T cell.

According to specific embodiments, the T cell is a memory T cell. Non-limiting examples of memory T cells include effector memory CD4+ T cells with a CD3+/CD4+/CD45RA-/CCR7- phenotype, central memory CD4+ T cells with a CD3+/CD4+/CD45RA-/CCR7+ phenotype, effector memory CD8+ T cells with a CD3+/CD8+ CD45RA-/CCR7-phenotype and central memory CD8+ T cells with a CD3+/CD8+ CD45RA-/CCR7+ phenotype.

According to specific embodiments, the T cell is an NKT cell.

As used herein the term "NKT cell" refers to a specialized T cell that express a variety of molecular markers that are typically associated with NK cells, such as NK1.1. NKT cells include NK1.1+ and NK1.1-, as well as CD4+, CD4-, CD8+ and CD8-cells.

According to other specific embodiments, the T cells is not an NKT cell.

According to specific embodiments, the T cell is a human T cell.

According to specific embodiments, the T cell is of a healthy subject.

According to specific embodiments, the T cell is of a subject suffering from a pathology (e.g. cancer).

According to specific embodiments, the T cell expresses an endogenous CD3.

According to specific embodiments, the T cell expresses an exogenous CD3.

Methods of obtaining T cells are well known in the art. Thus, for examples, PBMCs can be isolated by drawing whole blood from a subject and collection in a container containing an anti-coagulant (e.g. heparin or citrate); and apheresis. According to other specific embodiments, the T cells are obtained from a tissue comprising cells associated with a pathology. Methods for obtaining a tissue sample from a subject are well known in the art and include e.g. biopsy, surgery or necropsy and preparing a single cell suspension thereof. Following, according to specific embodiments, the T cell is enriched or purified from the peripheral blood or from the single cell suspension. There are several methods and reagents known to those skilled in the art for purifying T cells such as leukapheresis, sedimentation, density gradient centrifugation (e.g. ficoll), centrifugal elutriation, fractionation, chemical lysis of e.g. red blood cells (e.g. by ACK), selection of T cells using cell surface markers (using e.g. FACS sorter or magnetic cell separation techniques such as are commercially available e.g. from Invitrogen, Stemcell Technologies, Cellpro, Advanced Magnetics, or Miltenyi Biotec.), and depletion of other cell types by methods such as eradication (e.g. killing) with specific antibodies or by affinity based purification based on negative selection (using e.g. magnetic cell separation techniques, FACS sorter and/or capture ELISA labeling). Such methods are described for example in THE HANDBOOK OF EXPERIMENTAL IMMUNOLOGY, Volumes 1 to 4, (D.N. Weir, editor) and FLOW CYTOMETRY AND CELL SORTING (A. Radbruch, editor, Springer Verlag, 2000).

The T cells of some embodiments of the invention is modified (or engineered or transduced) to upregulate or downregulate expression of a gene of interest (e.g. endogenous TCR, PD1, TGFBR1, B2M, CTLA4).

According to specific embodiments, the T cell does not expresses an endogenous TCR.

Thus, according to specific embodiments, the method further comprising downregulating expression of the endogenous TCR prior to introducing to the T cell the at least one polynucleotide encoding the TCR disclosed herein.

Methods of downregulating expression of a gene of interest e.g. an endogenous TCR are well known in the art [see for example WO2019222275, WO2015143224, US20190388472; the contents of which are incorporated by reference in their entireties] and include targeted homologous recombination, site specific recombinases, PB transposases and genome editing by engineered nucleases [Zinc finger nucleases (ZFNs), transcription-activator like effector nucleases (TALENs), CRISPR/Cas system and constant shRNA]. Agents for introducing nucleic acid alterations to a TCR can be designed publically available sources or obtained commercially from Transposagen, Addgene and Sangamo Biosciences. A non-limiting example of a method of downregulating an endogenous TCR is described in details in the Examples section which follows.

According to specific embodiments, the T cells can be freshly isolated, stored e.g., cryopreserved (i.e. frozen) at e.g. liquid nitrogen temperature at any stage for long periods of time (e.g., months, years) for future use; and cell lines.

Methods of cryopreservation are commonly known by one of ordinary skill in the art and are disclosed e.g. in International Patent Application Publication Nos. WO2007054160 and WO 2001039594 and US Patent Application Publication No. US20120149108.

According to specific embodiments, the T cells can be stored in a cell bank or a depository or storage facility.

Consequently, the present teachings further suggest the use of the T cells and the methods disclosed herein as, but not limited to, a source for adoptive cells therapies.

Thus, according to an aspect of the present invention, the T cells disclosed herein are for use in adoptive cell therapy.

The T cells used according to specific embodiments of the present invention may be autologous or non-autologous; they can be syngeneic or non-syngeneic: allogeneic or xenogeneic to the subject; each possibility represents a separate embodiment of the present invention.

According to specific embodiments, the cells are autologous to the subject.

According to specific embodiments, the cells are non-autologous to the subject.

According to specific embodiments, the cells are allogeneic to the subject.

As the T cells of some embodiments do not express an endogenous TCR and express a TCR or TCR complex devoid of an antigen-binding domain, the T cells of some embodiments do not stimulate a graft versus host disease in a non-autologous subj ect.

According to specific embodiments, the T cells described herein are cultured, expanded and/or activated ex-vivo prior to administration to the subject.

Methods of culturing, expanding and activating T cells are well known to the skilled in the art. For example, the T cells of some embodiments may be expanded ex-*vivo* in the presence of an anti-CD3 antibody, an anti-CD28 antibody, anti-CD3 and anti-CD28 coated beads (such as the CD3CD28 MACSiBeads obtained from Miltenyi Biotec) with or without IL-2.

Since the TCR or TCR complex disclosed herein are devoid of an antigen-binding domain, the T cells of some embodiments of the invention may be administered to a subject in combination with a therapeutic composition directed for binding a target cell (e.g. a pathological cell) on the one hand and the TCR complex on the other hand, thereby activating the T cell towards the target cell.

Thus, according to an aspect of the present invention, there is provided a method of treating a disease associated with a pathological cell in a subject in need thereof, the method comprising administering to the subject a therapeutically effective amount of the T cell disclosed herein; and a therapeutic composition capable of binding the pathological cell and the TCR complex, thereby treating the disease in the subject.

According to an additional or an alternative aspect of the present invention, there is provided the T cell disclosed herein; and a therapeutic composition capable of binding a pathological cell and the TCR complex, for use in treating a disease associated with the pathological cell in a subject in need thereof.

As used herein, the term "subject" includes mammals, preferably human beings at any age and of any gender. According to specific embodiments, the term "subject" refers to a subject who suffers from the pathology (disease, disorder or medical condition). According to specific embodiments, this term encompasses individuals who are at risk to develop the pathology.

As used herein the term "treating" refers to curing, reversing, attenuating, alleviating, minimizing, suppressing or halting the deleterious effects of a disease or disorder (e.g. cancer). Those of skill in the art will understand that various methodologies and assays can be used to assess the development of a pathology, and similarly, various methodologies and assays may be used to assess the reduction, remission or regression of a pathology (e.g. a malignancy), as discussed below.

As used herein, the term "preventing" refers to keeping a disease, disorder or condition from occurring in a subject who may be at risk for the disease, but has not yet been diagnosed as having the disease.

As used herein the phrase, "disease associated with a pathological cell" means that pathologic cells drive onset and/or progression of the disease.

According to specific embodiments, the disease can benefit from modulating immune cells.

As used herein the phrase "a disease that can benefit from modulating immune cells" refers to diseases in which the subject's immune response activity may be sufficient to at least ameliorate symptoms of the disease or delay onset of symptoms, however for any reason the activity of the subject's immune response in doing so is less than optimal.

According to specific embodiments, the disease can benefit from activating immune cells.

Non-limiting examples of diseases that can benefit from activating immune cells include hyper-proliferative diseases, diseases associated with immune suppression, immunosuppression caused by medication (e.g. mTOR inhibitors, calcineurin inhibitor, steroids) and infections.

According to specific embodiments, the disease comprises an infection.

As used herein, the term "infection" or "infectious disease" refers to a disease induced by a pathogen. Specific examples of pathogens include, viral pathogens, bacterial pathogens e.g., intracellular mycobacterial pathogens (such as, for example, Mycobacterium tuberculosis), intracellular bacterial pathogens (such as, for example, Listeria monocytogenes), or intracellular protozoan pathogens (such as, for example, Leishmania and Trypanosoma).

Specific types of viral pathogens causing infectious diseases include, but are not limited to, retroviruses, circoviruses, parvoviruses, papovaviruses, adenoviruses, herpesviruses, iridoviruses, poxviruses, hepadnaviruses, picornaviruses, caliciviruses, togaviruses, flaviviruses, reoviruses, orthomyxoviruses, paramyxoviruses, rhabdoviruses, bunyaviruses, coronaviruses, arenaviruses, and filoviruses.

Specific examples of viral infections which may be treated according to specific embodiments of the present invention include, but are not limited to, human immunodeficiency virus (HIV)-induced acquired immunodeficiency syndrome (AIDS), influenza, rhinoviral infection, viral meningitis, Epstein-Barr virus (EBV) infection, hepatitis A, B or C virus infection, measles, papilloma virus infection/warts, cytomegalovirus (CMV) infection, Herpes simplex virus infection, yellow fever, Ebola virus infection, rabies, etc.

According to specific embodiments, the disease comprises a hyper-proliferative disease.

According to specific embodiments, the hyper-proliferative disease comprises sclerosis, fibrosis, Idiopathic pulmonary fibrosis, psoriasis, systemic sclerosis/scleroderma, primary biliary cholangitis, primary sclerosing cholangitis, liver fibrosis, prevention of radiation-induced pulmonary fibrosis, myelofibrosis or retroperitoneal fibrosis.

According to other specific embodiments, the hyper-proliferative disease comprises cancer.

Thus, according to specific embodiments the pathological cell is a cancerous cell.

Cancers which may be treated by some embodiments of the invention can be any solid or non-solid tumor, cancer metastasis and/or a pre-cancer.

According to specific embodiments, the cancer is a malignant cancer.

Examples of cancer include but are not limited to, carcinoma, blastoma, sarcoma and lymphoma. More particular examples of such cancers include, but are not limited to, tumors of the gastrointestinal tract (colon carcinoma, rectal carcinoma, colorectal carcinoma, colorectal cancer, colorectal adenoma, hereditary nonpolyposis type 1, hereditary nonpolyposis type 2, hereditary nonpolyposis type 3, hereditary nonpolyposis type 6; colorectal cancer, hereditary nonpolyposis type 7, small and/or large bowel carcinoma, esophageal carcinoma, tylosis with esophageal cancer, stomach carcinoma, pancreatic carcinoma, pancreatic endocrine tumors), endometrial carcinoma, dermatofibrosarcoma protuberans, gallbladder carcinoma, Biliary tract tumors, prostate cancer, prostate adenocarcinoma, renal cancer (e.g., Wilms' tumor type 2 or type 1), liver cancer (e.g., hepatoblastoma, hepatocellular carcinoma, hepatocellular cancer), bladder cancer, embryonal rhabdomyosarcoma, germ cell tumor, trophoblastic tumor, testicular germ cells tumor, immature teratoma of ovary, uterine, epithelial ovarian, sacrococcygeal tumor, choriocarcinoma, placental site trophoblastic tumor, epithelial adult tumor, ovarian carcinoma, serous ovarian cancer, ovarian sex cord tumors, cervical carcinoma, uterine cervix carcinoma, small-cell and non-small cell lung carcinoma, nasopharyngeal, breast carcinoma (e.g., ductal breast cancer, invasive intraductal breast cancer, sporadic ; breast cancer, susceptibility to breast cancer, type 4 breast cancer, breast cancer-1, breast cancer-3; breast-ovarian cancer), squamous cell carcinoma (e.g., in head and neck), neurogenic tumor, astrocytoma, ganglioblastoma, neuroblastoma, lymphomas (e.g., Hodgkin's disease, non-Hodgkin's lymphoma, B cell, Burkitt, cutaneous T cell, histiocytic, lymphoblastic, T cell, thymic), gliomas, adenocarcinoma, adrenal tumor, hereditary adrenocortical carcinoma, brain malignancy (tumor), various other carcinomas (e.g., bronchogenic large cell, ductal, Ehrlich-Lettre ascites, epidermoid, large cell, Lewis lung, medullary, mucoepidermoid, oat cell, small cell, spindle cell, spinocellular, transitional cell, undifferentiated, carcinosarcoma, choriocarcinoma, cystadenocarcinoma), ependimoblastoma, epithelioma, erythroleukemia (e.g., Friend, lymphoblast), fibrosarcoma, giant cell tumor, glial tumor, glioblastoma (e.g., multiforme, astrocytoma), glioma hepatoma, heterohybridoma, heteromyeloma, histiocytoma, hybridoma (e.g., B cell), hypernephroma, insulinoma, islet tumor, keratoma, leiomyoblastoma, leiomyosarcoma, leukemia (e.g., acute lymphatic, acute lymphoblastic, acute lymphoblastic pre-B cell, acute lymphoblastic T cell leukemia, acute - megakaryoblastic, monocytic, acute myelogenous, acute myeloid, acute myeloid with eosinophilia, B cell, basophilic, chronic myeloid, chronic, B cell, eosinophilic, Friend, granulocytic or myelocytic, hairy cell, lymphocytic, megakaryoblastic, monocytic, monocytic-macrophage, myeloblastic, myeloid, myelomonocytic, plasma cell, pre-B cell, promyelocytic, subacute, T cell, lymphoid neoplasm, predisposition to myeloid malignancy, acute nonlymphocytic leukemia), lymphosarcoma, melanoma, mammary tumor, mastocytoma, medulloblastoma, mesothelioma, metastatic tumor, monocyte tumor, multiple myeloma, myelodysplastic syndrome, myeloma, nephroblastoma, nervous tissue glial tumor, nervous tissue neuronal tumor, neurinoma, neuroblastoma, oligodendroglioma, osteochondroma, osteomyeloma, osteosarcoma (e.g., Ewing's), papilloma, transitional cell, pheochromocytoma, pituitary tumor (invasive), plasmacytoma, retinoblastoma, rhabdomyosarcoma, sarcoma (e.g., Ewing's, histiocytic cell, Jensen, osteogenic, reticulum cell), schwannoma, subcutaneous tumor, teratocarcinoma (e.g., pluripotent), teratoma, testicular tumor, thymoma and trichoepithelioma, gastric cancer, fibrosarcoma, glioblastoma multiforme; multiple glomus tumors, Li-Fraumeni syndrome, liposarcoma, lynch cancer family syndrome II, male germ cell tumor, mast cell leukemia, medullary thyroid, multiple meningioma, endocrine neoplasia myxosarcoma, paraganglioma, familial nonchromaffin, pilomatricoma, papillary, familial and sporadic, rhabdoid predisposition syndrome, familial, rhabdoid tumors, soft tissue sarcoma, and Turcot syndrome with glioblastoma.

According to specific embodiments, the cancer is a pre-malignant cancer.

Pre-cancers are well characterized and known in the art (refer, for example, to Berman JJ. and Henson DE., 2003. Classifying the pre-cancers: a metadata approach. BMC Med Inform Decis Mak. 3:8). Examples of pre-cancers include, but are not limited to, acquired small pre-cancers, acquired large lesions with nuclear atypia, precursor lesions occurring with inherited hyperplastic syndromes that progress to cancer, and acquired diffuse hyperplasias and diffuse metaplasias. Non-limiting examples of small pre-cancers include HGSIL (High grade squamous intraepithelial lesion of uterine cervix), AIN (anal intraepithelial neoplasia), dysplasia of vocal cord, aberrant crypts (of colon), PIN (prostatic intraepithelial neoplasia).

Non-limiting examples of acquired large lesions with nuclear atypia include tubular adenoma, AILD (angioimmunoblastic lymphadenopathy with dysproteinemia), atypical meningioma, gastric polyp, large plaque parapsoriasis, myelodysplasia, papillary transitional cell carcinoma *in-situ,* refractory anemia with excess blasts, and Schneiderian papilloma. Non-limiting examples of precursor lesions occurring with inherited hyperplastic syndromes that progress to cancer include atypical mole syndrome, C cell adenomatosis and MEA. Non-limiting examples of acquired diffuse hyperplasias and diffuse metaplasias include Paget's disease of bone and ulcerative colitis.

According to specific embodiments, the cancer is selected from the group consisting of lymphoma, leukemia, glioblastoma, colon cancer, gastric cancer, pancreatic cancer, ovarian cancer, lung cancer and skin cancer.

According to specific embodiments, the disease can benefit from inhibiting immune cells.

According to specific embodiments, the disease is an autoimmune disease. Such autoimmune diseases include, but are not limited to, cardiovascular diseases, rheumatoid diseases, glandular diseases, gastrointestinal diseases, cutaneous diseases, hepatic diseases, neurological diseases, muscular diseases, nephric diseases, diseases related to reproduction, connective tissue diseases and systemic diseases.

Examples of autoimmune cardiovascular diseases include, but are not limited to atherosclerosis (Matsuura E. et al., Lupus. 1998;7 Suppl 2:S135), myocardial infarction (Vaarala O. Lupus. 1998;7 Suppl 2:S132), thrombosis (Tincani A. et al., Lupus 1998;7 Suppl 2:S107-9), Wegener's granulomatosis, Takayasu's arteritis, Kawasaki syndrome (Praprotnik S. et al., Wien Klin Wochenschr 2000 Aug 25;112 (15-16):660), anti-factor VIII autoimmune disease (Lacroix-Desmazes S. et al., Semin Thromb Hemost.2000;26 (2):157), necrotizing small vessel vasculitis, microscopic polyangiitis, Churg and Strauss syndrome, pauci-immune focal necrotizing and crescentic glomerulonephritis (Noel LH. Ann Med Interne (Paris). 2000 May;151 (3):178), antiphospholipid syndrome (Flamholz R. et al., J Clin Apheresis 1999; 14 (4):171), antibody-induced heart failure (Wallukat G. et al., Am J Cardiol. 1999 Jun 17;83 (12A):75H), thrombocytopenic purpura (Moccia F. Ann Ital Med Int. 1999 Apr-Jun;14 (2):114; Semple JW. et al., Blood 1996 May 15;87 (10):4245), autoimmune hemolytic anemia (Efremov DG. et al., Leuk Lymphoma 1998 Jan;28 (3-4):285; Sallah S. et al., Ann Hematol 1997 Mar;74 (3):139), cardiac autoimmunity in Chagas' disease (Cunha-Neto E. et al., J Clin Invest 1996 Oct 15;98 (8):1709) and anti-helper T lymphocyte autoimmunity (Caporossi AP. et al., Viral Immunol 1998; 11 (1):9).

Examples of autoimmune rheumatoid diseases include, but are not limited to rheumatoid arthritis (Krenn V. et al., Histol Histopathol 2000 Jul;15 (3):791; Tisch R, McDevitt HO. Proc Natl Acad Sci units S A 1994 Jan 18;91 (2):437) and ankylosing spondylitis (Jan Voswinkel et al., Arthritis Res 2001; 3 (3): 189).

Examples of autoimmune glandular diseases include, but are not limited to, pancreatic disease, Type I diabetes, thyroid disease, Graves' disease, thyroiditis, spontaneous autoimmune thyroiditis, Hashimoto's thyroiditis, idiopathic myxedema, ovarian autoimmunity, autoimmune anti-sperm infertility, autoimmune prostatitis and Type I autoimmune polyglandular syndrome. diseases include, but are not limited to autoimmune diseases of the pancreas, Type 1 diabetes (Castano L. and Eisenbarth GS. Ann. Rev. Immunol. 8:647; Zimmet P. Diabetes Res Clin Pract 1996 Oct;34 Suppl:S125), autoimmune thyroid diseases, Graves' disease (Orgiazzi J. Endocrinol Metab Clin North Am 2000 Jun;29 (2):339; Sakata S. et al., Mol Cell Endocrinol 1993 Mar;92 (1):77), spontaneous autoimmune thyroiditis (Braley-Mullen H. and Yu S, J Immunol 2000 Dec 15;165 (12):7262), Hashimoto's thyroiditis (Toyoda N. et al., Nippon Rinsho 1999 Aug;57 (8):1810), idiopathic myxedema (Mitsuma T. Nippon Rinsho. 1999 Aug;57 (8):1759), ovarian autoimmunity (Garza KM. et al., J Reprod Immunol 1998 Feb;37 (2):87), autoimmune anti-sperm infertility (Diekman AB. et al., Am J Reprod Immunol. 2000 Mar;43 (3):134), autoimmune prostatitis (Alexander RB. et al., Urology 1997 Dec;50 (6):893) and Type I autoimmune polyglandular syndrome (Hara T. et al., Blood. 1991 Mar 1;77 (5): 1127).

Examples of autoimmune gastrointestinal diseases include, but are not limited to, chronic inflammatory intestinal diseases (Garcia Herola A. et al., Gastroenterol Hepatol. 2000 Jan;23 (1):16), celiac disease (Landau YE. and Shoenfeld Y. Harefuah 2000 Jan 16;138 (2):122), colitis, ileitis and Crohn's disease.

Examples of autoimmune cutaneous diseases include, but are not limited to, autoimmune bullous skin diseases, such as, but are not limited to, pemphigus vulgaris, bullous pemphigoid and pemphigus foliaceus.

Examples of autoimmune hepatic diseases include, but are not limited to, hepatitis, autoimmune chronic active hepatitis (Franco A. et al., Clin Immunol Immunopathol 1990 Mar;54 (3):382), primary biliary cirrhosis (Jones DE. Clin Sci (Colch) 1996 Nov;91 (5):551; Strassburg CP. et al., Eur J Gastroenterol Hepatol. 1999 Jun;11 (6):595) and autoimmune hepatitis (Manns MP. J Hepatol 2000 Aug;33 (2):326).

Examples of autoimmune neurological diseases include, but are not limited to, multiple sclerosis (Cross AH. et al., J Neuroimmunol 2001 Jan 1;112 (1-2):1), Alzheimer's disease (Oron L. et al., J Neural Transm Suppl. 1997;49:77), myasthenia gravis (Infante AJ. And Kraig E, Int Rev Immunol 1999; 18 (1-2):83; Oshima M. et al., Eur J Immunol 1990 Dec;20 (12):2563), neuropathies, motor neuropathies (Kornberg AJ. J Clin Neurosci. 2000 May;7 (3):191); Guillain-Barre syndrome and autoimmune neuropathies (Kusunoki S. Am J Med Sci. 2000 Apr;319 (4):234), myasthenia, Lambert-Eaton myasthenic syndrome (Takamori M. Am J Med Sci. 2000 Apr;319 (4):204); paraneoplastic neurological diseases, cerebellar atrophy, paraneoplastic cerebellar atrophy and stiff-man syndrome (Hiemstra HS. et al., Proc Natl Acad Sci units S A 2001 Mar 27;98 (7):3988); non-paraneoplastic stiff man syndrome, progressive cerebellar atrophies, encephalitis, Rasmussen's encephalitis, amyotrophic lateral sclerosis, Sydeham chorea, Gilles de la Tourette syndrome and autoimmune polyendocrinopathies (Antoine JC. and Honnorat J. Rev Neurol (Paris) 2000 Jan;156 (1):23); dysimmune neuropathies (Nobile-Orazio E. et al., Electroencephalogr Clin Neurophysiol Suppl 1999;50:419); acquired neuromyotonia, arthrogryposis multiplex congenita (Vincent A. et al., Ann N Y Acad Sci. 1998 May 13;841:482), neuritis, optic neuritis (Soderstrom M. et al., J Neurol Neurosurg Psychiatry 1994 May;57 (5):544) and neurodegenerative diseases.

Examples of autoimmune muscular diseases include, but are not limited to, myositis, autoimmune myositis and primary Sjogren's syndrome (Feist E. et al., Int Arch Allergy Immunol 2000 Sep;123 (1):92) and smooth muscle autoimmune disease (Zauli D. et al., Biomed Pharmacother 1999 Jun;53 (5-6):234).

Examples of autoimmune nephric diseases include, but are not limited to, nephritis and autoimmune interstitial nephritis (Kelly CJ. J Am Soc Nephrol 1990 Aug;1 (2):140).

Examples of autoimmune diseases related to reproduction include, but are not limited to, repeated fetal loss (Tincani A. et al., Lupus 1998;7 Suppl 2:S107-9).

Examples of autoimmune connective tissue diseases include, but are not limited to, ear diseases, autoimmune ear diseases (Yoo TJ. et al., Cell Immunol 1994 Aug;157 (1):249) and autoimmune diseases of the inner ear (Gloddek B. et al., Ann N Y Acad Sci 1997 Dec 29;830:266).

Examples of autoimmune systemic diseases include, but are not limited to, systemic lupus erythematosus (Erikson J. et al., Immunol Res 1998;17 (1-2):49) and systemic sclerosis (Renaudineau Y. et al., Clin Diagn Lab Immunol. 1999 Mar;6 (2):156); Chan OT. et al., Immunol Rev 1999 Jun;169:107).

According to specific embodiments, the disease is graft rejection disease.

Examples of diseases associated with transplantation of a graft include, but are not limited to, graft rejection, chronic graft rejection, subacute graft rejection, hyperacute graft rejection, acute graft rejection and graft versus host disease.

According to specific embodiments, the disease is an allergic disease.

Examples of allergic diseases include, but are not limited to, asthma, hives, urticaria, pollen allergy, dust mite allergy, venom allergy, cosmetics allergy, latex allergy, chemical allergy, drug allergy, insect bite allergy, animal dander allergy, stinging plant allergy, poison ivy allergy and food allergy.

As mentioned, according to specific embodiments, the T cells are administered to the subject in combination with a therapeutic composition specific for the pathological cell [e.g. binds an antigen overexpressed or solely expressed by a pathologic (e.g. cancerous) cell as compared to a non-pathologic cell] on the one hand and capable of binding the TCR complex on the other hand.

According to specific embodiments, the therapeutic composition binds CD3.

According to specific embodiments, the therapeutic composition binds the constant or the hinge region of the TCR.

According to specific embodiments, the therapeutic composition binds a heterologous tag fused to the TCR complex or a tag bound to the TCR complex. Non-limiting Examples of such tags are known in the art and are further described in details hereinabove.

The administration of the T cells and the administration of the therapeutic composition can be effected in the same route or in separate routes.

The administration of the T cells may be prior to, following or concomitant with the therapeutic.

Multiple rounds of administration of the T cells and/or the therapeutic composition can be administered.

According to specific embodiments, multiple distinct therapeutic compositions specific for the pathological cell (e.g. targeting different antigens) and capable of binding the TCR complex are provided to the subject.

Therapeutic compositions capable of binding a pathological cell and the TCR complex are known in the art and include, but not limited to, antibodies such as bispecific and tri-specific antibodies.

The term "antibody" as used herein includes intact molecules as well as functional fragments thereof (that are capable of binding to an epitope of an antigen).

As used herein, the term "epitope" refers to any antigenic determinant on an antigen to which the paratope of an antibody binds. Epitopic determinants usually consist of chemically active surface groupings of molecules such as amino acids or carbohydrate side chains and usually have specific three dimensional structural characteristics, as well as specific charge characteristics.

According to a specific embodiment, the antibody fragments include, but are not limited to, single chain, Fab, Fab' and F(ab')₂ fragments, Fd, Fcab, Fv, dsFv, scFvs, diabodies, minibodies, nanobodies, Fab expression library or single domain molecules such as VH and VL that are capable of binding to an epitope of the antigen in an HLA restricted manner.

According to specific embodiments, the therapeutic compositions is at least a bispecific antibody.

As used herein, the term "bi-specific antibody" refers to an antibody having two distinct antigen-binding moieties, wherein the first binding moiety has affinity to the TCR complex and the second binding moiety has affinity for an antigen distinct from the TCR complex. According to specific embodiments, the bi-specific antibody binds the TCR complex on the one hand and an antigen expressed by a pathological cell (e.g. cancerous cell) on the other hand.

Methods of producing bi-specific antibodies are known in the art and disclosed for examples in US Patent Numbers 4,474,893, 5,959,084, US and 7,235,641, 7,183,076, U.S. Publication Number 20080219980 and International Publication Numbers WO 2010/115589, WO2013150043 and WO2012118903 all incorporated herein by their entirety; and include, for example, chemical cross-linking (Brennan, et al., Science 229,81 (1985); Raso, et al., J. BioI. Chern. 272, 27623 (1997)), disulfide exchange, production of hybrid-hybridomas (quadromas), by transcription and translation to produce a single polypeptide chain embodying a bi-specific antibody, or by transcription and translation to produce more than one polypeptide chain that can associate covalently to produce a bi-specific antibody. The contemplated bi-specific antibody can also be made entirely by chemical synthesis.

Hence, according to specific embodiments, the therapeutic antibody comprises at least one arm that binds an antigen overexpressed or solely expressed by a pathological cell and one arm comprising an antibody moiety that binds the TCR complex.

Selection of the therapeutic antibody used is well within the capability of those skilled in the art, and depends on the type of the disease and the antigens expressed by the pathologic cells associated with the pathology.

According to specific embodiments, the therapeutic composition comprises an anti-CD3 antibody.

According to specific embodiments, the anti-CD3 antibody moiety is of an antibody selected from the group consisting of diL2K, TR66, OKT3 UCHT1, humanized UHCT1 and F6A.

According to specific embodiments, the anti-CD3 antibody moiety is of an antibody selected from the group consisting of diL2K, TR66 and OKT3.

According to specific embodiments, the therapeutic antibody binds an antigen expressed by cancerous cells. Non-limiting examples of such antigens include CD19, EGFR, HER2, MUC-1, CA-125, mesothelin, ROR1, GPC3, PSCA, CD133, CD70, EpCAM, CEA, CAIX, CD171, GD2, Tn-MUC1, EGFRvIII, ADGRE2, CD33, CD123, CCR1, CLEC12A, LILRB2, BCMA, CD138, gp100 and the antigens disclosed in Q He et al. ()2019) J Hematol Oncol 12, 99, the contents of which are fully incorporated herein by reference.

According to some embodiments of the invention, the therapeutic antibody is selected from the group consisting of Blinatumomab, AMG 330, AMG701, Orlotamab, AMG420, CC-93269 APVO436, JNJ-63709178, AMG757, MT110, Tebentafusp., Odronextamab, RG6007, RG6194, RG6232, RG7828 mosunetuzumab, RG7802 cibisatamab, cevostamab, Glofitamab and IMMTAC (e.g. RG6290).

According to specific embodiments, the pathological cells expresses CD19 and the therapeutic composition comprises Blinatumomab.

According to specific embodiments, the pathological cell expresses EpCAM and said therapeutic composition comprises MT110.

According to specific embodiments, the pathological cell expresses CD20 and said therapeutic composition comprises Odronextamab.

According to specific embodiments, the pathological cell expresses HLA-A2-WT1 and said therapeutic composition comprises RG6007.

According to specific embodiments, the pathological cell expresses HER2 and said therapeutic composition comprises RG6194.

According to specific embodiments, the pathological cell expresses TYRP1 and said therapeutic composition comprises RG6232.

According to specific embodiments, the pathological cell expresses MAGE-A4 and said therapeutic composition comprises RG6290. According to specific embodiments, the therapeutic composition comprises an anti-TCR antibody.

According to specific embodiments, the anti-TCR antibody moiety is of an antibody clone selected from the group consisting of IP26, WT31, T10B9, 8A3, 3A8, Jovi-1.

According to specific embodiments, the T cells and the therapeutic compositions capable of binding the pathological cell and the TCR complex can be administered to a subject in combination with other established or experimental therapeutic regimen to treat a disease associated with pathologic cells (e.g. cancer) including, but not limited to analgesics, chemotherapeutic agents, radiotherapeutic agents, cytotoxic therapies (conditioning), hormonal therapy and other treatment regimens (e.g., surgery) which are well known in the art.

The T cells disclosed herein and/or the therapeutic compositions capable of binding the pathological cell and the TCR complex disclosed herein can be administered to the subject *per se,* or in a pharmaceutical composition where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the T cells and/or the therapeutic compositions capable of binding the pathological cell and the TCR complex accountable for the biological effect.

Thus, according to specific embodiments, the T cells are the active ingredient in the formulation.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition, which is incorporated herein by reference.

Suitable routes of administration may, for example, include oral, rectal, transmucosal, especially transnasal, intestinal or parenteral delivery, including intramuscular, intradermal, subcutaneous and intramedullary injections as well as intrathecal, direct intraventricular, intracardiac, e.g., into the right or left ventricular cavity, into the common coronary artery, intravenous, intraperitoneal, intranasal, or intraocular injections.

Conventional approaches for drug delivery to the central nervous system (CNS) include: neurosurgical strategies (e.g., intracerebral injection or intracerebroventricular infusion); molecular manipulation of the agent (e.g., production of a chimeric fusion protein that comprises a transport peptide that has an affinity for an endothelial cell surface molecule in combination with an agent that is itself incapable of crossing the BBB) in an attempt to exploit one of the endogenous transport pathways of the BBB; pharmacological strategies designed to increase the lipid solubility of an agent (e.g., conjugation of water-soluble agents to lipid or cholesterol carriers); and the transitory disruption of the integrity of the BBB by hyperosmotic disruption (resulting from the infusion of a mannitol solution into the carotid artery or the use of a biologically active agent such as an angiotensin peptide). However, each of these strategies has limitations, such as the inherent risks associated with an invasive surgical procedure, a size limitation imposed by a limitation inherent in the endogenous transport systems, potentially undesirable biological side effects associated with the systemic administration of a chimeric molecule comprised of a carrier motif that could be active outside of the CNS, and the possible risk of brain damage within regions of the brain where the BBB is disrupted, which renders it a suboptimal delivery method.

Alternately, one may administer the pharmaceutical composition in a local rather than systemic manner, for example, via injection of the pharmaceutical composition directly into a tissue region of a patient.

According to a specific embodiment, the T cells of the invention or the pharmaceutical composition comprising same is administered via an IV route.

Pharmaceutical compositions of some embodiments of the invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with some embodiments of the invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to some embodiments of the invention are conveniently delivered in the form of an aerosol spray presentation from a pressurized pack or a nebulizer with the use of a suitable propellant, e.g., dichlorodifluoromethane, trichlorofluoromethane, dichlorotetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of some embodiments of the invention may also be formulated in rectal compositions such as suppositories or retention enemas, using, e.g., conventional suppository bases such as cocoa butter or other glycerides.

Alternative embodiments include depots providing sustained release or prolonged duration of activity of the active ingredient in the subject, as are well known in the art.

Pharmaceutical compositions suitable for use in context of some embodiments of the invention include compositions wherein the active ingredients are contained in an amount effective to achieve the intended purpose. More specifically, a therapeutically effective amount means an amount of active ingredients effective to prevent, alleviate or ameliorate symptoms of a disorder (e.g., cancer) or prolong the survival of the subject being treated.

Determination of a therapeutically effective amount is well within the capability of those skilled in the art, especially in light of the detailed disclosure provided herein.

For any preparation used in the methods of the invention, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide levels of the active ingredient are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of some embodiments of the invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as is further detailed above.

According to another aspect of the present invention there is provided an article of manufacture comprising a packaging material packaging the T cell disclosed herein; and a therapeutic composition capable of binding a pathological cell and the TCR complex.

According to specific embodiments, the article of manufacture is identified for the treatment of a disease associated with a pathologic cell (e.g. cancer).

According to specific embodiments, the T cells and the therapeutic composition are packaged in separate containers.

According to specific embodiments, the T cells and the therapeutic composition are packaged in a co-formulation.

As used herein the term "about" refers to ± 10 %

The terms "comprises", "comprising", "includes", "including", "having" and their conjugates mean "including but not limited to".

The term "consisting of' means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

When reference is made to particular sequence listings, such reference is to be understood to also encompass sequences that substantially correspond to its complementary sequence as including minor sequence variations, resulting from, e.g., sequencing errors, cloning errors, or other alterations resulting in base substitution, base deletion or base addition, provided that the frequency of such variations is less than 1 in 50 nucleotides, alternatively, less than 1 in 100 nucleotides, alternatively, less than 1 in 200 nucleotides, alternatively, less than 1 in 500 nucleotides, alternatively, less than 1 in 1000 nucleotides, alternatively, less than 1 in 5,000 nucleotides, alternatively, less than 1 in 10,000 nucleotides.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion. Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### MATERIALS AND METHODS

***Cell culture and Reagents*** - K562, Raji and 293T cell lines (JCRB) were cultured as recommended by the supplier. All cell lines were authenticated by short tandem repeat (STR) profiling using PowerPlex16 HS kit (Promega). Cell number and viability were estimated using hemocytometer and trypan blue exclusion assay, respectively. Research grade of Blinatumomab and MT110 were purchased from Oak BioSciences, Inc (CA, USA).

***Generation of TCR negative T cells*** - Human peripheral blood mononuclear cells (PBMCs) cells were purified from peripheral blood samples of healthy donors, using Ficoll-Hypaque density gradient centrifugation. Extracted PBMCs were activated and expanded with OKT3 (Biolegend, Cat. No 317301), in 24-well suspension plates in 4Cell^{®} Nutri-T Media (Sartorius, 05-11F2001-1K) supplemented with 500 U / ml of IL-2 (Peprotech, 200-02) and antibiotics. 72 hours following activation cells were electroporated using AMAXA Nucleofector (Lonza) with SpCas9 RNPs using Alt-R CRISPR-Cas9 System and sgRNA that target the human constant alpha region (IDT, Coralville). Electroporated cells were cultured in 4Cell^{®} Nutri-T Media supplemented with 500 U / ml of IL-2 and antibiotics. Three days following electroporation, most of the CD3+/TCR+ T cells were negative for the constant alpha chain (> 85 %) determined by anti-CD3 flow antibody (PE-Cy7 OKT3 Biolegend, Cat No. 317333). The negative cells were further enriched by magnetic anti-CD3 beads (Miltenyi Biotec, Cat No. 130-050-101), following manufacturers' instructions, to reach 95%-99.9% CD3-/TCR- cells (Figure 3A).

***DNA constructs and cloning* -**An RTV-020 retroviral vector (Cell Biolabs Inc. San Diego, CA) was used. For each construct, the amino acid sequence was optimized (IDT, Coralville), each sequence was designed to express a polypeptide encoding a different TCR alpha and/or beta polypeptides, V5-furin-P2A sequence (S Yang, et al. 2008) and EGFP or truncated NGFR (dNGFR) as indicated. Schematic representations of the designed constructs are shown in Figure 2. The final sequences were ordered as gBlocks^{®} (IDT, Coralville). gBlocks^{®} were amplified using proper primers and High fidelity PrimeSTAR GXL DNA Polymerase (Cat No. R050B, TAKARA) then digested with BamHI-HF and XhoI (NEB) ligated into the retroviral vector. When indicated, 3 mutations were introduced in the transmembrane domain of the alpha constant chain: S116L, G119V and F120L (LVL). When indicated cysteines were introduced in both the alpha and beta chains (Cys): T48C in the alpha chain and S57C in the beta chain. When indicated (marked as mm), several mutations were introduced in the extracellular domain of the constant alpha and beta domains, as follows: constant alpha mutations P90S, E91D, S92V, S93P; constant beta mutations: E18K, S22A, F133I, E/V136A, Q139H. Sanger sequencing was used to validate cloned vectors.

***Virus preparation and transduction procedure*** - Viral particles were generated by transient transfection of 293T cells with the contracted retroviral vector using CMV-gagPol and CMV-VSVG (Cell Biolabs, Inc.). CRISPR-edited TCR negative T cells were seeded on tissue culture plates coated with Retronectin (TaKaRa Bio) and the concentrated viral particles were added. Cells were incubated with the viral preparations for 7 hours, and then the medium was replaced with 4Cell^{®} Nutri-T Media supplemented with 500 U / ml of IL-2 and antibiotics.

***Flow cytometry analysis*** - Cells were stained with OKT3 Anti-CD3 -APC (Biolegend), following manufacturers' instructions. Results were obtained using Gallios^{™} and Cytoflex Flow Cytometers (Beckman Coulter, Inc.). Flow cytometry results were analyzed using FlowJo v10 software.

### EXAMPLE 1

### GENERATION OF A TRUNCATED T CELL RECEPTOR (TCR)

The present inventors have devised a novel engineered TCR that is devoid of the variable regions of the TCR alpha and beta chains. This TCR, referred to herein as "blunt truncated TCR (BluT)", is presented as part of the TCR complex on the cell surface (Figure 1A) and can serve as a TCR that does not recognize any antigen through the MHC machinery, thus preventing the induction of GvHD even in cases of mismatch pairing with the endogenous TCR alpha or beta chains (Figure 1B).

To this end, several constructs encoding TCR alpha and/or beta chains devoid of their variable regions were designed (Figure 2, amino acid sequences are provided in SEQ ID NOs:2-3 and 26-32; nucleic acid sequences are provided in SEQ ID NOs: 33-41), packed into retroviruses and used to infect TCR negative T cells (formed by CRISPR targeting of the TCR alpha chain) (Figures 3A-B). EGFP was used as a marker of stable infection. As shown in Figure 3B and Table 1 hereinbelow, introducing a vector encoding only a truncated alpha chain or only a truncated beta chain did not result in presentation of a TCR complex on the cell surface (manifested by no expression of CD3 on the surface). However, introducing a vector encoding a truncated alpha chain comprising an additional cysteine and a truncated beta chain comprising an additional cysteine resulted in presentation of a TCR complex on the cell surface (manifested by re-expression of CD3 on the surface).

**Table 1:**

| SEQ ID NO: | Construct name | %EGFP | % of CD3+ in EGFP- | % of CD3+ in EGFP+ | CD3 MFI Ration between EGFP+/EGFP- |
|---|---|---|---|---|---|
| | Non infected control | 0 | 95 | 0 | 0 |
| 26/34 | EGFP-P2A-TRAC(Cys)-P2A-TRBC1(Cys) | 30 | 4.53 | 40.5 | 7.537 |
| 27/35 | EGFP-P2A-TRAC(Cys)-FurinV5P2A-TRBC1(Cys) | 27 | 4.3 | 48 | 9.85 |
| 2/33 | EGFP-P2A-TRAC(Cys,LVL)-P2A-TRBC1(Cys) | 31 | 4.45 | 48.4 | 11.29 |
| 28/36 | EGFP-P2A-TRAC(Cys,LVL)-FurinV5P2A-TRBC1(Cys) | 18 | 3.9 | 68 | 34.5 |
| 29/37 | EGFP-P2A-mmTRAC-FurinV5P2A-mmTRBC 1 | 20 | 4.25 | 45 | 20.8 |
| 30/38 | EGFP-P2A-mmTRAC(Cys)-FurinV5P2A-mmTRBC1(Cys) | 15 | 3.33 | 55.733 | 27.05 |
| 31/39 | EGFP-P2A-mmTRAC(LVL)-FurinV5P2A-mmTRBC 1 | 27 | 4.73 | 69.63 | 31.033 |
| 32/40 | EGFP-P2A-mmTRAC(Cys,LVL)-FurinV5P2A-mmTRBC1(Cys) | 24 | 3.61 | 67.9 | 35.25 |

### EXAMPLE 2

### T CELLS EXPRESSING BluT TCR IN COMBINATION WITH A BISPECIFIC T CELL ENGAGER INDUCE EFFECTIVE TUMOR CELL LYSIS

As the therapeutic strategy of some embodiments is based on infusing to a subject T cells expressing the BluT along with a bi-specific antibody binding the TCR complex on one hand (e.g. CD3) and a cancer antigen on the other hand, the cytotoxic activity of the transduced T cells is evaluated in-vitro on CD19+ Raji cells and/or EGFR+ K562 cells in combination with the anti-CD19 bispecific T-cell engager (BiTE)- Blinatumomab and/or the anti EPCAM BiTE- MT110. T cell activation is determined by expression of CD107 and secretion of cytokines such as IFNγ, TNFα, IL-2. Cytotoxic activity is determined by reduction in CD19 or EGFR percentage in comparison with non-treated controls. For positive selection of transduced T cells with BluT, dNGFR is used as a selection marker (instead of the EGFP described in Figure 2 and Example 1 hereinabove).

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

### REFERENCES

### (other references are cited throughout the application)

1. MacKay M, Afshinnekoo E, Rub J, Hassan C, Khunte M, Baskaran N, et al. The therapeutic landscape for cells engineered with chimeric antigen receptors. Nat Biotechnol [Internet]. 2020 [cited 2020 Jan 20]; Available from: http://www(dot)ncbi(dot)nlm(dot)nih(dot)gov/pubmed/31907405
2. Sadelain M, Rivière I, Riddell S. Therapeutic T cell engineering. Nature [Internet]. Nature Publishing Group; 2017;545:423-31. Available from: http ://dx(dot)doi(dot)org/10(dot) 103 8/nature223 95
3. Fesnak AD, June CH, Levine BL. Engineered T cells: The promise and challenges of cancer immunotherapy. Nat Rev Cancer [Internet]. Nature Publishing Group; 2016;16:566-81. Available from: http://dx(dot)doi(dot)org/10(dot)1038/nrc(dot)2016(dot)97
4. Kershaw MH, Westwood JA, Darcy PK. Gene-engineered T cells for cancer therapy. Nat Rev Cancer [Internet]. Nature Publishing Group; 2013;13:525-41. Available from: http://dx(dot)doi(dot)org/10(dot)1038/nrc3565
5. Safarzadeh Kozani P, Safarzadeh Kozani P, Rahbarizadeh F, Khoshtinat Nikkhoi S. Strategies for Dodging the Obstacles in CAR T Cell Therapy [Internet]. Front. Oncol. Frontiers Media S.A.; 2021 [cited 2021 May 27]. page 924. Available from: www(dot)frontiersin(dot)org
6. Goebeler ME, Bargou RC. T cell-engaging therapies - BiTEs and beyond. Nat Rev Clin Oncol [Internet]. Springer US; 2020;17:418-34. Available from: http://dx(dot)doi(dot)org/10(dot)1038/s41571-020-0347-5
7. Rajat Bannerji, MD, PhD, John N. Allan, MD, Jon E. Arnason, MD, Jennifer R. Brown, MD, PhD, Ranjana Advani, MD, Stephen M. Ansell, MD, PhD, Susan M. O'Brien, MD, Johannes Duell, MD, Peter Martin, FRCPC, MD, MS, Robin M. Joyce, MD, Jingjin Li, PhD, Dina M. M. Odronextamab (REGN1979), a Human CD20 x CD3 Bispecific Antibody, Induces Durable, Complete Responses in Patients with Highly Refractory B-Cell Non-Hodgkin Lymphoma, Including Patients Refractory to CAR T Therapy. 2020. Available from: https://ash(dot)confex(dot)com/ash/2020/webprogram/Paper136659 .html
8. Ahamadi-Fesharaki R, Fateh A, Vaziri F, Solgi G, Siadat SD, Mahboudi F, et al. Single-Chain Variable Fragment-Based Bispecific Antibodies: Hitting Two Targets with One Sophisticated Arrow. Mol Ther - Oncolytics [Internet]. Elsevier Ltd.; 2019;14:38-56. Available from: https://doi(dot)org/10(dot)1016/j(dot)omto(dot)2019(dot)02(dot)004
9. Garber K. Driving T-cell immunotherapy to solid tumors. Nat Biotechnol. Nature Publishing Group; 2018;36:215-9.
10. Slaney CY, Wang P, Darcy PK, Kershaw MH. CARs versus biTEs: A comparison between T cell-redirection strategies for cancer treatment [Internet]. Cancer Discov. American Association for Cancer Research Inc.; 2018 [cited 2021 May 27]. page 924-34. Available from: www(dot)aacrjournals(dot)org
11. Chandran SS, Klebanoff CA. T cell receptor-based cancer immunotherapy: Emerging efficacy and pathways of resistance. Immunol Rev. 2019;290:127-47.
12. Gudipati V, Rydzek J, Doel-Perez I, Gonçalves VDR, Scharf L, Königsberger S, et al. Inefficient CAR-proximal signaling blunts antigen sensitivity. Nat Immunol. 2020;21:848-56.
13. Helsen CW, Hammill JA, Lau VWC, Mwawasi KA, Afsahi A, Bezverbnaya K, et al. The chimeric TAC receptor co-opts the T cell receptor yielding robust anti-tumor activity without toxicity. Nat Commun [Internet]. Springer US; 2018;9:1-13. Available from: http://dx(dot)doi(dot)org/10(dot)1038/s41467-018-05395-y
14. Baeuerle PA, Ding J, Patel E, Thorausch N, Horton H, Gierut J, et al. Synthetic TRuC receptors engaging the complete T cell receptor for potent anti-tumor response. Nat Commun [Internet]. Springer US; 2019;10:1-12. Available from: http://dx(dot)doi(dot)org/10(dot)1038/s41467-019-10097-0
15. Lee YG, Chu H, Lu Y, Leamon CP, Srinivasarao M, Putt KS, et al. Regulation of CAR T cell-mediated cytokine release syndrome-like toxicity using low molecular weight adapters. Nat Commun [Internet]. Springer US; 2019;10:1-11. Available from: http://dx(dot)doi(dot)org/10(dot)1038/s41467-019-10565-7
16. Ghorashian S, Kramer AM, Onuoha S, Wright G, Bartram J, Richardson R, et al. Enhanced CAR T cell expansion and prolonged persistence in pediatric patients with ALL treated with a low-affinity CD19 CAR. Nat. Med. Nature Publishing Group; 2019. page 1408-14.
17. Benjamin R, Graham C, Yallop D, Jozwik A, Mirci-Danicar OC, Lucchini G, et al. Genome-edited, donor-derived allogeneic anti-CD 19 chimeric antigen receptor T cells in paediatric and adult B-cell acute lymphoblastic leukaemia: results of two phase 1 studies. Lancet. 2020;396:1885-94.
18. Alderton GK. Immunotherapy: Engineered T cells for all. Nat. Rev. Cancer. Nature Publishing Group; 2017. page 206-7.
19. Van Loenen MM, De Boer R, Amir AL, Hagedoorn RS, Volbeda GL, Willemze R, et al. Mixed T cell receptor dimers harbor potentially harmful neoreactivity. Proc Natl Acad Sci USA. 2010;107:10972-7.
20. Mack M, Gruber R, Schmidt S, Riethmüller G, Kufer P. Biologic properties of a bispecific single-chain antibody directed against 17-1A (EpCAM) and CD3: tumor cell-dependent T cell stimulation and cytotoxic activity. J Immunol. 1997;158.
21. Exley M, Terhorst C, Wileman T. Structure, assembly and intracellular transport of the T cell receptor for antigen. Semin Immunol. 1991;3:283-97.
22. Garfall AL, Dancy EK, Cohen AD, Hwang WT, Fraietta JA, Davis MM, et al. T-cell phenotypes associated with effective CAR T-cell therapy in postinduction vs relapsed multiple myeloma. Blood Adv. 2019;3:2812-5.
23. Lamers CHJ, Sleijfer S, Vulto AG, Kruit WHJ, Kliffen M, Debets R, et al. Treatment of metastatic renal cell carcinoma with autologous T-lymphocytes genetically retargeted against carbonic anhydrase IX: first clinical experience. J Clin Oncol [Internet]. 2006 [cited 2020 Jan 20];24:e20-2. Available from: http://www(dot)ncbi(dot)nlm(dot)nih(dot)gov/pubmed/16648493
24. Morgan RA, Yang JC, Kitano M, Dudley ME, Laurencot CM, Rosenberg SA. Case report of a serious adverse event following the administration of t cells transduced with a chimeric antigen receptor recognizing ERBB2. Mol Ther. 2010;18:843-51.
25. Linette GP, Stadtmauer EA, Maus M V., Rapoport AP, Levine BL, Emery L, et al. Cardiovascular toxicity and titin cross-reactivity of affinity-enhanced T cells in myeloma and melanoma. Blood. 2013;122:863-71.
26. Morgan RA, Chinnasamy N, Abate-Daga D, Gros A, Robbins PF, Zheng Z, et al. Cancer regression and neurological toxicity following anti-MAGE-A3 TCR gene therapy. J Immunother. 2013;36:133-51.
27. Brudno JN, Kochenderfer JN. Toxicities of chimeric antigen receptor T cells: Recognition and management. Blood. American Society of Hematology; 2016. page 3321-30.
28. Maude SL, Barrett D, Teachey DT, Grupp SA. Managing cytokine release syndrome associated with novel T cell-engaging therapies. Cancer J. (United States). Lippincott Williams and Wilkins; 2014. page 119-22.
29. Grupp SA, Kalos M, Barrett D, Aplenc R, Porter DL, Rheingold SR, et al. Chimeric Antigen Receptor-Modified T Cells for Acute Lymphoid Leukemia. N Engl J Med [Internet]. 2013 [cited 2020 Jan 20];368:1509-18. Available from: http://www(dot)nejm(dot)org/doi/10(dot)1056/NEJMoa1215134
30. Fry TJ, Shah NN, Orentas RJ, Stetler-Stevenson M, Yuan CM, Ramakrishna S, et al. CD22-targeted CAR T cells induce remission in B-ALL that is naive or resistant to CD19-targeted CAR immunotherapy. Nat Med. Nature Publishing Group; 2018;24:20-8.
31. Choi BD, Yu X, Castano AP, Bouffard AA, Schmidts A, Larson RC, et al. CART cells secreting BiTEs circumvent antigen escape without detectable toxicity. Nat Biotechnol. Nature Publishing Group; 2019;37:1049-58.
32. Liu Y, Liu G, Wang J, Zheng ZY, Jia L, Rui W, et al. Chimeric STAR receptors using TCR machinery mediate robust responses against solid tumors. Sci Transl Med. 2021;13.
33. Yang S, Cohen CJ, Peng PD, Zhao Y, Cassard L, Yu Z, et al. Development of optimal bicistronic lentiviral vectors facilitates high-level TCR gene expression and robust tumor cell recognition. Gene Ther [Internet]. Nature Publishing Group; 2008 [cited 2021 May 28];15:1411-23. Available from: www(dot)nature(dot)com/gt
34. Haga-Friedman A, Horovitz-Fried M, Cohen CJ. Incorporation of Transmembrane Hydrophobic Mutations in the TCR Enhance Its Surface Expression and T Cell Functional Avidity. J Immunol. 2012;188:5538-46.
35. Jin BY, Campbell TE, Draper LM, Stevanovic S, Weissbrich B, Yu Z, et al. Engineered T cells targeting E7 mediate regression of human papillomavirus cancers in a murine model. JCI insight. 2018;3.
36. Kuball J, Hauptrock B, Malina V, Antunes E, Voss RH, Wolfl M, et al. Increasing functional avidity of TCR- Redirected T cells by removing defined N- glycosylation sites in the TCR constant domain. J Exp Med. 2009;206:463-75.
37. Cohen CJ, Li YF, El-Gamil M, Robbins PF, Rosenberg SA, Morgan RA. Enhanced antitumor activity of T cells engineered to express T-cell receptors with a second disulfide bond. Cancer Res. 2007;67:3898-903.
38. Boulter JM, Glick M, Todorov PT, Baston E, Sami M, Rizkallah P, et al. Stable, soluble T-cell receptor molecules for crystallization and therapeutics. Protein Eng. 2003;16:707-11.
39. Kuball J, Dossett ML, Wolfl M, Ho WY, Voss RH, Fowler C, et al. Facilitating matched pairing and expression of TCR chains introduced into human T cells. Blood. 2007;109:2331-8.
40. Froning K, Maguire J, Sereno A, Huang F, Chang S, Weichert K, et al. Computational stabilization of T cell receptors allows pairing with antibodies to form bispecifics. Nat Commun [Internet]. Springer US; 2020;11:1-14. Available from: http://dx(dot)doi(dot)org/10(dot)1038/s41467-020-16231-7
41. Sommermeyer D, Uckert W. Minimal Amino Acid Exchange in Human TCR Constant Regions Fosters Improved Function of TCR Gene-Modified T Cells. J Immunol. 2010; 184:6223-31.
42. Brischwein K, Schlereth B, Guller B, Steiger C, Wolf A, Lutterbuese R, et al. MT110: A novel bispecific single-chain antibody construct with high efficacy in eradicating established tumors. Mol Immunol [Internet]. Mol Immunol; 2006 [cited 2021 May 30];43:1129-43. Available from: https://pubmed(dot)ncbi(dot)nlm(dot)nih(dot)gov/16139892/

## Claims

1. A T cell receptor (TCR) complex comprising a TCR comprising a TCR alpha polypeptide and a TCR beta polypeptide, wherein said TCR is devoid of an antigen-binding domain, and a CD3 polypeptide devoid of a heterologous antigen-binding domain; and wherein said TCR complex is capable of being presented on a surface of a T cell expressing same.

2. A T cell receptor (TCR) comprising a human TCR alpha polypeptide and a human TCR beta polypeptide, wherein said TCR is devoid of an antigen-binding domain, and wherein said TCR alpha and beta polypeptides comprise amino acid modifications enabling presentation of said TCR as a TCR complex on a surface of a T cell expressing same.

3. The TCR complex of claim 1 or said TCR of claim 2, wherein said TCR comprises a heterologous dimerizing moiety.

4. The TCR complex or the TCR of claim 3, wherein said heterologous dimerizing moiety comprises a cysteine residue at each of said TCR alpha polypeptide and said TCR beta polypeptide.

5. The TCR complex of any one of claims 1 and 3-4, wherein said TCR alpha polypeptide and said TCR beta polypeptide comprise a human TCR alpha polypeptide and a human TCR beta polypeptide.

6. The TCR complex or TCR of any one of claims 1-4, wherein said TCR alpha polypeptide and said TCR beta polypeptide comprise a chimeric human and murine TCR alpha polypeptide and a chimeric human and murine TCR beta polypeptide.

7. The TCR complex or TCR of any one of claims 1-2, wherein said TCR alpha polypeptide comprises an amino acid sequence selected form the group consisting of SEQ ID NO: 14, 17 and 20-23.

8. The TCR complex of any one of claims 1 and 7, wherein said TCR beta polypeptide comprises an amino acid sequence selected form the group consisting of SEQ ID NO: 15-16, 18-19 and 24-25.

9. At least one polynucleotide encoding the TCR of any one of claims 2-8.

10. A transduced T cell expressing the TCR complex or TCR of any one of claims 1-8 or the at least one polynucleotide of claim 9.

11. A method of expressing a TCR in a T cell, the method comprising introducing into a T cell the at least one polynucleotide of claim 9, under conditions which allow expression of said TCR.

12. The T cell or the method of any one of claims 10-11, wherein said T cell does not express an endogenous TCR.

13. The method of claim 12, further comprising downregulating expression of said endogenous TCR prior to said introducing.

14. The T cell of any one of claims 10 and 12; and a therapeutic composition capable of binding a pathological cell and said TCR complex, for use in treating a disease associated with said pathological cell in a subject in need thereof.

15. An article of manufacture comprising a packaging material packaging the T cell of any one of claims 10 and 12; and a therapeutic composition capable of binding a pathological cell and said TCR complex.
